# EUROPEAN PATENT APPLICATION

(11) **EP 4 049 683 A1**
(43) Date of publication of application: **31.08.2022**
(21) Application number: 21217892.5
(22) Date of filing: 01.05.2017
(51) Int. Cl.: A61K 48/00, C12N 15/00, C12N 15/11, C12N 15/86, C12N 15/861, C12N 5/02

(54) **EVASION OF NEUTRALIZING ANTIBODIES BY A RECOMBINANT ADENO-ASSOCIATED VIRUS**

(30) Priority: 29.04.2016 US 201662330002 P
(62) Divisional of application: 17790633.6
(71) Applicant: Adverum Biotechnologies, Inc., Redwood City, CA 94063 (US)
(72) Inventor: KERAVALA, Annahita, Redwood City, 94063 (US); CHALBERG, Thomas W, Redwood City, 94063 (US)
(74) Representative: Schiweck Weinzierl Koch Patentanwälte Partnerschaft mbB

(57) **Abstract**

Provided herein are methods for expression of a transgene in the presence of neutralizing antibodies by administering a recombinant adeno-associated virus (rAAV) virion in an amount capable of evading the neutralizing antibodies.

SEQUENCE LISTING

<110>  Adverum Biotechnologies, Inc.

<120>  EVASION OF NEUTRALIZING ANTIBODIES BY A RECOMBINANT
       ADENO-ASSOCIATED VIRUS

<130>  COL16564PCTEPD1

<150>  US 62/330,002
<151>  2016-04-29

<160>  29

<170>  PatentIn version 3.5

<210>  1
<211>  11
<212>  PRT
<213>  Artificial Sequence

<220>
<223>  7m8 insersertion sequence motif

<220>
<221>  MISC_FEATURE
<222>  (1)..(2)
<223>  Xaa is Ala, Leu, Gly, Ser, Thr or absent

<220>
<221>  MISC_FEATURE
<222>  (3)..(3)
<223>  Xaa is Leu, Asn or Lys

<220>
<221>  MISC_FEATURE
<222>  (4)..(4)
<223>  Xaa is Gly, Glu, Ala or Asp

<220>
<221>  MISC_FEATURE
<222>  (5)..(5)
<223>  Xaa is Glu, Thr, Gly or Pro

<220>
<221>  MISC_FEATURE
<222>  (6)..(6)
<223>  Xaa is Thr, Ile, Gln or Lys

<220>
<221>  MISC_FEATURE
<222>  (7)..(7)
<223>  Xaa is Thr or Ala

<220>
<221>  MISC_FEATURE
<222>  (8)..(8)
<223>  Xaa is Arg, Asn or Thr

<220>
<221>  MISC_FEATURE

<222>  (9)..(9)
<223>  Xaa is Pro, Asn or absent

<220>
<221>  MISC_FEATURE
<222>  (10)..(11)
<223>  Xaa is Ala, Leu, Gly, Ser, Thr or absent

<400>  1

Xaa Xaa Xaa Xaa Xaa Xaa Xaa Xaa Xaa Xaa Xaa
1               5                   10


<210>  2
<211>  11
<212>  PRT
<213>  Artificial Sequence

<220>
<223>  7m8 insersertion sequence motif


<220>
<221>  MISC_FEATURE
<222>  (1)..(2)
<223>  Xaa is Ala, Leu, Gly, Ser, Thr or absent

<220>
<221>  MISC_FEATURE
<222>  (3)..(6)
<223>  Xaa is any amino acid

<220>
<221>  MISC_FEATURE
<222>  (10)..(11)
<223>  Xaa is Ala, Leu, Gly, Ser, Thr or absent

<400>  2

Xaa Xaa Xaa Xaa Xaa Xaa Thr Arg Pro Xaa Xaa
1               5                   10


<210>  3
<211>  11
<212>  PRT
<213>  Artificial Sequence

<220>
<223>  7m8 insersertion sequence motif


<220>
<221>  MISC_FEATURE
<222>  (1)..(2)
<223>  Xaa is Ala, Leu, Gly, Ser, Thr or absent

<220>
<221>  MISC_FEATURE
<222>  (3)..(3)
<223>  Xaa is Leu, Asn or absent

<220>
<221> MISC_FEATURE
<222> (4)..(4)
<223> Xaa is Gly, Glu or absent

<220>
<221> MISC_FEATURE
<222> (5)..(5)
<223> Xaa is Glu or Thr

<220>
<221> MISC_FEATURE
<222> (6)..(6)
<223> Xaa is Thr or Ile

<220>
<221> MISC_FEATURE
<222> (10)..(11)
<223> Xaa is Ala, Leu, Gly, Ser, Thr or absent

<400> 3

Xaa Xaa Xaa Xaa Xaa Xaa Thr Arg Pro Xaa Xaa
1               5                   10


<210> 4
<211> 11
<212> PRT
<213> Artificial Sequence

<220>
<223> 7m8 insersertion sequence motif


<220>
<221> MISC_FEATURE
<222> (1)..(2)
<223> Xaa is Ala, Leu, Gly, Ser, Thr or absent

<220>
<221> MISC_FEATURE
<222> (3)..(3)
<223> Xaa is Lys or absent

<220>
<221> MISC_FEATURE
<222> (4)..(4)
<223> Xaa is Ala or Asp

<220>
<221> MISC_FEATURE
<222> (5)..(5)
<223> Xaa is Gly or Pro

<220>
<221> MISC_FEATURE
<222> (6)..(6)
<223> Xaa is Gln or Lys

<220>
<221> MISC_FEATURE
<222> (7)..(7)

<223>  Xaa is Thr or Ala

<220>
<221>  MISC_FEATURE
<222>  (8)..(8)
<223>  Xaa is Asn or Thr

<220>
<221>  MISC_FEATURE
<222>  (9)..(9)
<223>  Xaa is Asn or absent

<220>
<221>  MISC_FEATURE
<222>  (10)..(11)
<223>  Xaa is Ala, Leu, Gly, Ser, Thr or absent

<400>  4

Xaa Xaa Xaa Xaa Xaa Xaa Xaa Xaa Xaa Xaa Xaa
1               5                   10


<210>  5
<211>  11
<212>  PRT
<213>  Artificial Sequence

<220>
<223>  7m8 insersertion sequence motif


<220>
<221>  MISC_FEATURE
<222>  (1)..(2)
<223>  Xaa is Ala, Leu, Gly, Ser, Thr or absent

<220>
<221>  MISC_FEATURE
<222>  (3)..(3)
<223>  Xaa is a positively charged amino acid or an uncharged amino
       acid; or is selected from Leu, Asn, Arg, Ala, Ser, and Lys; or
       absent

<220>
<221>  MISC_FEATURE
<222>  (4)..(4)
<223>  Xaa is a negatively charged amino acid or an uncharged amino
       acid; or is selected from Gly, Glu, Ala, Val, Thr, and Asp

<220>
<221>  MISC_FEATURE
<222>  (5)..(5)
<223>  Xaa is a negatively charged amino acid or an uncharged amino
       acid; or is selected from Glu, Thr, Gly, Asp, or Pro

<220>
<221>  MISC_FEATURE
<222>  (6)..(6)
<223>  Xaa is selected from Thr, Ile, Gly, Lys, Asp, and Gln

<220>
<221>  MISC_FEATURE

<222>  (7)..(7)
<223>  Xaa is a polar amino acid, an alcohol (an amino acid having a
       free hydroxyl group), or a hydrophobic amino acid; or is selected
       from Thr, Ser, Val, and Ala

<220>
<221>  MISC_FEATURE
<222>  (8)..(8)
<223>  Xaa is a positively charged amino acid or an uncharged amino
       acid; or is selected from Arg, Val, Lys, Pro, Thr, and Asn

<220>
<221>  MISC_FEATURE
<222>  (9)..(9)
<223>  Xaa is a positively charged amino acid or an uncharged amino
       acid; or is selected from Pro, Gly, Phe, Asn, and Arg; or is
       absent

<220>
<221>  MISC_FEATURE
<222>  (10)..(11)
<223>  Xaa is Ala, Leu, Gly, Ser, Thr or absent

<400>  5

Xaa Xaa Xaa Xaa Xaa Xaa Xaa Xaa Xaa Xaa Xaa
1               5                   10


<210>  6
<211>  7
<212>  PRT
<213>  Artificial Sequence

<220>
<223>  Synthesized 7m8 insertion peptide

<400>  6

Leu Gly Glu Thr Thr Arg Pro
1               5


<210>  7
<211>  7
<212>  PRT
<213>  Artificial Sequence

<220>
<223>  Synthesized 7m8 insertion peptide

<400>  7

Asn Glu Thr Ile Thr Arg Pro
1               5


<210>  8
<211>  7
<212>  PRT
<213>  Artificial Sequence

<220>

<223>  Synthesized 7m8 insertion peptide

<400>  8

Lys Ala Gly Gln Ala Asn Asn
1               5


<210>  9
<211>  7
<212>  PRT
<213>  Artificial Sequence

<220>
<223>  Synthesized 7m8 insertion peptide

<400>  9

Lys Asp Pro Lys Thr Thr Asn
1               5


<210>  10
<211>  7
<212>  PRT
<213>  Artificial Sequence

<220>
<223>  Synthesized 7m8 insertion peptide

<400>  10

Lys Asp Thr Asp Thr Thr Arg
1               5


<210>  11
<211>  7
<212>  PRT
<213>  Artificial Sequence

<220>
<223>  Synthesized 7m8 insertion peptide

<400>  11

Arg Ala Gly Gly Ser Val Gly
1               5


<210>  12
<211>  7
<212>  PRT
<213>  Artificial Sequence

<220>
<223>  Synthesized 7m8 insertion peptide

<400>  12

Ala Val Asp Thr Thr Lys Phe
1               5

<210>  13
<211>  7
<212>  PRT
<213>  Artificial Sequence

<220>
<223>  Synthesized 7m8 insertion peptide

<400>  13

Ser Thr Gly Lys Val Pro Asn
1               5


<210>  14
<211>  10
<212>  PRT
<213>  Artificial Sequence

<220>
<223>  Synthesized 7m8 insertion peptide

<400>  14

Leu Ala Leu Gly Glu Thr Thr Arg Pro Ala
1               5                   10


<210>  15
<211>  10
<212>  PRT
<213>  Artificial Sequence

<220>
<223>  Synthesized 7m8 insertion peptide

<400>  15

Leu Ala Asn Glu Thr Ile Thr Arg Pro Ala
1               5                   10


<210>  16
<211>  10
<212>  PRT
<213>  Artificial Sequence

<220>
<223>  Synthesized 7m8 insertion peptide

<400>  16

Leu Ala Lys Ala Gly Gln Ala Asn Asn Ala
1               5                   10


<210>  17
<211>  10
<212>  PRT
<213>  Artificial Sequence

<220>

<223>  Synthesized 7m8 insertion peptide

<400>  17

Leu Ala Lys Asp Pro Lys Thr Thr Asn Ala
1               5                   10


<210>  18
<211>  10
<212>  PRT
<213>  Artificial Sequence

<220>
<223>  Synthesized 7m8 insertion peptide

<400>  18

Leu Ala Lys Asp Thr Asp Thr Thr Arg Ala
1               5                   10


<210>  19
<211>  10
<212>  PRT
<213>  Artificial Sequence

<220>
<223>  Synthesized 7m8 insertion peptide

<400>  19

Leu Ala Arg Ala Gly Gly Ser Val Gly Ala
1               5                   10


<210>  20
<211>  10
<212>  PRT
<213>  Artificial Sequence

<220>
<223>  Synthesized 7m8 insertion peptide

<400>  20

Leu Ala Ala Val Asp Thr Thr Lys Phe Ala
1               5                   10


<210>  21
<211>  10
<212>  PRT
<213>  Artificial Sequence

<220>
<223>  Synthesized 7m8 insertion peptide

<400>  21

Leu Ala Ser Thr Gly Lys Val Pro Asn Ala
1               5                   10

<210>  22
<211>  10
<212>  PRT
<213>  Artificial Sequence

<220>
<223>  Synthesized 7m8 insertion peptide

<400>  22

Ala Ala Leu Gly Glu Thr Thr Arg Pro Ala
1               5                   10


<210>  23
<211>  10
<212>  PRT
<213>  Artificial Sequence

<220>
<223>  Synthesized 7m8 insertion peptide

<400>  23

Ala Ala Asn Glu Thr Ile Thr Arg Pro Ala
1               5                   10


<210>  24
<211>  10
<212>  PRT
<213>  Artificial Sequence

<220>
<223>  Synthesized 7m8 insertion peptide

<400>  24

Ala Ala Lys Ala Gly Gln Ala Asn Asn Ala
1               5                   10


<210>  25
<211>  10
<212>  PRT
<213>  Artificial Sequence

<220>
<223>  Synthesized 7m8 insertion peptide

<400>  25

Ala Ala Lys Asp Pro Lys Thr Thr Asn Ala
1               5                   10


<210>  26
<211>  9
<212>  PRT
<213>  Artificial Sequence

<220>

<223>  Synthesized 7m8 insertion peptide

<400>  26

Gly Leu Gly Glu Thr Thr Arg Pro Ala
1               5


<210>  27
<211>  9
<212>  PRT
<213>  Artificial Sequence

<220>
<223>  Synthesized 7m8 insertion peptide

<400>  27

Gly Asn Glu Thr Ile Thr Arg Pro Ala
1               5


<210>  28
<211>  9
<212>  PRT
<213>  Artificial Sequence

<220>
<223>  Synthesized 7m8 insertion peptide

<400>  28

Gly Lys Ala Gly Gln Ala Asn Asn Ala
1               5


<210>  29
<211>  9
<212>  PRT
<213>  Artificial Sequence

<220>
<223>  Synthesized 7m8 insertion peptide

<400>  29

Gly Lys Asp Pro Lys Thr Thr Asn Ala
1               5

## Description

### CROSS REFERENCE TO RELATED APPLICATIONS

This application claims the benefit of U.S. Provisional Application No. 62/330,002 filed April 29, 2016, the disclosure of which is hereby incorporated by reference herein in its entirety.

### BACKGROUND OF THE INVENTION

AAV-mediated gene therapy administered sub-retinally (SR) has been effective for some ocular diseases. Intravitreal (IVT) administration is less invasive and may be beneficial for a diseased, fragile retina. IVT delivery of AAV vectors may also be advantageous for more widespread retinal gene expression.

Available data suggests that neutralizing antibodies (nAbs) do not block transduction by AAV vectors following SR delivery due to ocular immune privilege. However, pre-existing nAbs may negatively impact transduction and gene expression following IVT delivery. Accordingly, there is a need in the art for methods for IVT delivery of AAV vectors that avoid nAbs and successfully transduce ocular cells.

### SUMMARY OF THE INVENTION

In one aspect, the present invention provides a method for providing a transgene encoding a gene product to an ocular cell in a subject, comprising administering to one or more sites within the eye of the subject an effective amount of a recombinant adeno-associated virus (rAAV) comprising said transgene, wherein the subject comprises neutralizing antibodies that specifically bind the rAAV, wherein the effective amount is an amount sufficient to at least partially evade the nAbs in the subject and transduce the ocular cell, and wherein the transduced ocular cell expresses the gene product. In particular embodiments, the cells comprise retinal cells. In particular embodiments, the rAAV is administered IVT.

In a related aspect, the present invention provides a method for treating an ocular disease or disorder in a subject in need thereof, comprising administering to one or more sites within the eye of the subject an effective amount of a recombinant adeno-associated virus (rAAV) comprising a transgene encoding a therapeutic gene product, wherein the subject comprises neutralizing antibodies that specifically bind the rAAV, wherein the effective amount is an amount sufficient to at least partially evade the nAbs in the subject and transduce ocular cells within the subject, and wherein the transduced ocular cells expresses the therapeutic gene product. In particular embodiments, the cells comprise retinal cells. In particular embodiments, the rAAV is administered IVT.

In another related aspect, the present invention provides a method for treating an ocular disease or disorder in a subject in need thereof, the method comprising: (a) administering to a first eye of the subject a first effective amount of a first recombinant adeno-associated virus (rAAV) comprising a first transgene encoding a first gene product; (b) waiting for a period of time; and (c) administering to a second eye of the subject a second effective amount of a second rAAV comprising a second transgene encoding a second gene product, wherein the first and second effective amounts are amounts sufficient to transduce cells of the eye, and wherein the transduced cells express the first and second gene products. In particular embodiments, the cells comprise retinal cells. In particular embodiments, the rAAV is administered IVT.

In particular embodiments of any aspect, the first rAAV and the second rAAV are the same serotype, the first rAAV and the second rAAV comprise the same capsid proteins, and/or the first rAAV and the second rAAV are AAV2.7m8. In particular embodiments of any aspect, the first rAAV and the second rAAV are different serotypes, the first rAAV and the second rAAV comprise different serotypes.

In particular embodiments, the period of time is selected from the following: at least one week, at least one month, at least three months, at least six months, at least one year, at least 18 months, at least two years, at least three years, or longer than three years. In certain embodiments, the subject is not administered a recombinant rAAV during the period of time.

In particular embodiments of any aspect, the first and second gene products are the same. In particular embodiments of any aspect, the first and second gene product are different. In certain embodiments, the first and/or second gene product is a therapeutic gene product. In certain embodiments, the first gene product and the second gene product are independently selected from: an anti-angiogenic polypeptide, a vascular endothelial growth factor (VEGF)-binding protein, an anti-VEGF agent or anti-VEGF protein, or an opsin protein.

In particular embodiments of any aspect, the ocular disease or disorder is glaucoma, retinitis pigmentosa, macular degeneration, retinoschisis, Leber's Congenital Amaurosis, diabetic retinopathy, achromotopsia, or color blindness. In certain embodiments, the ocular disease is macular degeneration, e.g., wet macular degeneration or dry macular degeneration.

In particular embodiments, the effective amount or first effective amount is at least about 1 × 10¹¹ vg, at least about 5 × 10¹¹ vg, at least about 1 × 10¹³ vg. In some embodiments, the effective amount or first effective amount comprises up to about 1 × 10¹¹ vector genomes of the first rAAV. In some embodiment, the effective amount or first effective amount comprises between about 1 × 10¹¹ and 5 × 10¹¹ vector genomes of the first rAAV.

In particular embodiments, the effective amount or second effective amount is at least about 1×10¹¹ vg, at least about 5×10¹¹ vg, or at least about 1×10¹³ vg.

In some embodiments, the first effective amount comprises up to about 1 × 10¹¹ vector genomes of the first rAAV, and the second effective amount comprises at least about 1×10¹¹ vector genomes of the second rAAV.

In certain embodiments of any of the aspects, the subject is not administered an immunosuppressant prior to, concurrent with, or following administration of the rAAV or first rAAV, whereas in other embodiments, the subject is administered an immunosuppressant prior to, concurrent with, or following administration of the rAAV or first rAAV.

In particular embodiments of any aspect, the administration is performed by ocular administration, retinal administration, subretinal administration and/or intravitreal administration. In particular embodiments, the administration is performed by ocular injection, retinal injection, subretinal injection and/or intravitreal injection.

In particular embodiments of any aspect, the rAAV is replication defective.

In certain aspects, disclosed herein are methods of transducing AAV2 vectors in the presence of neutralizing antibodies (nAbs) in a subject comprising administering to the subject a recombinant adeno -associated virus (rAAV) virion at an amount capable of at least partially evading nAbs in the subject. In some embodiments, the rAAV virion is AAV2.7m8. In some embodiments, the subject is a mammalian subject. In some embodiments, the amount of the rAAV virion administered is at least about 1×10¹¹ vg. In some embodiments, the amount of the rAAV virion administered is at least about 5×10¹¹ vg. In some embodiments, the amount of the rAAV virion administered is at least about 1 × 10¹³ vg. In some embodiments, the administration is an intravitreal injection.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

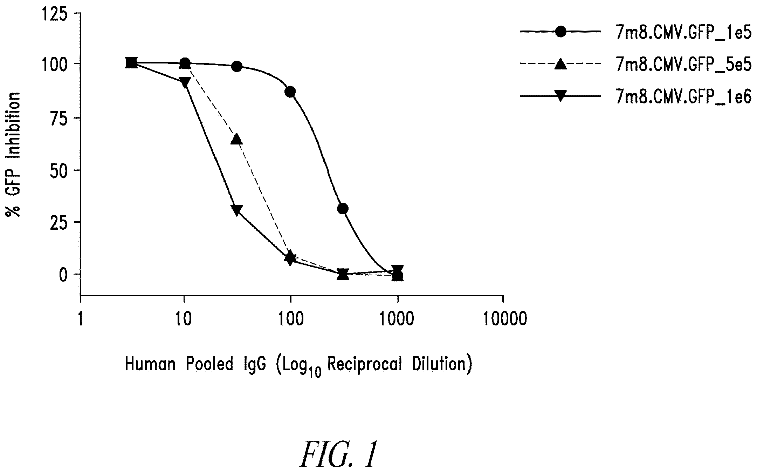

**FIG. 1****: Increasing vector doses can overcome inhibition by nAbs in vitro.** To detect the effect of increasing AAV2.7m8 titers on neutralization, an in vitro IVIg (Intravenous Immunoglobulin) assay was performed. Virus (MOIs in half-log increments from 1E5 to 5E6) was mixed in a 1:1 ratio with a 3-fold dilution series from 1:3-1:1000 of IVIg for 1 hour and added to 293T cells. These were incubated for 72 hours followed by assessment of % GFP inhibition.

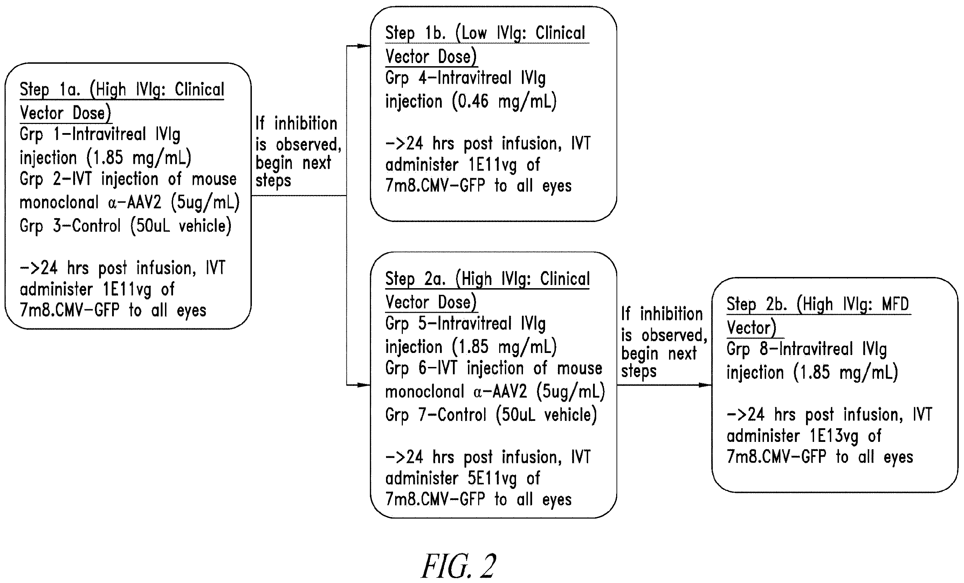

**FIG. 2****: In vivo study design overview.** African green monkeys (AGM) were used to model potentially relevant clinical conditions of neutralizing antibodies (nAbs). Varying levels of IVIg and AAV2.7m8 doses were tested to simulate the effect of pre-existing nAbs on IVT injected AAV2.7m8.

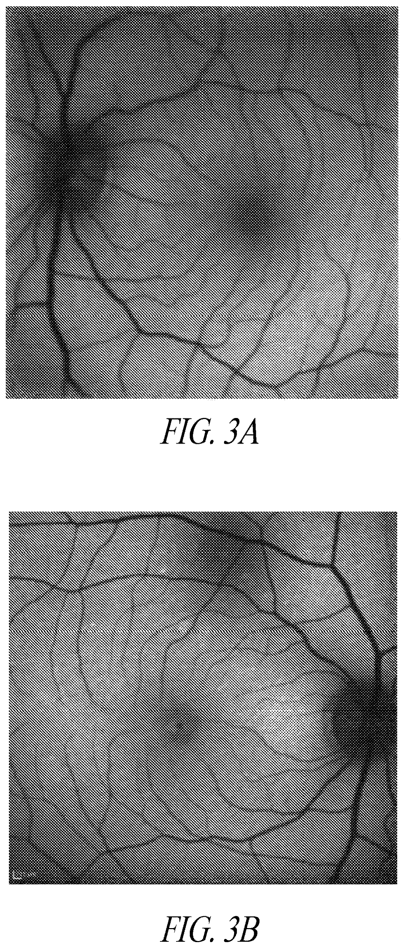

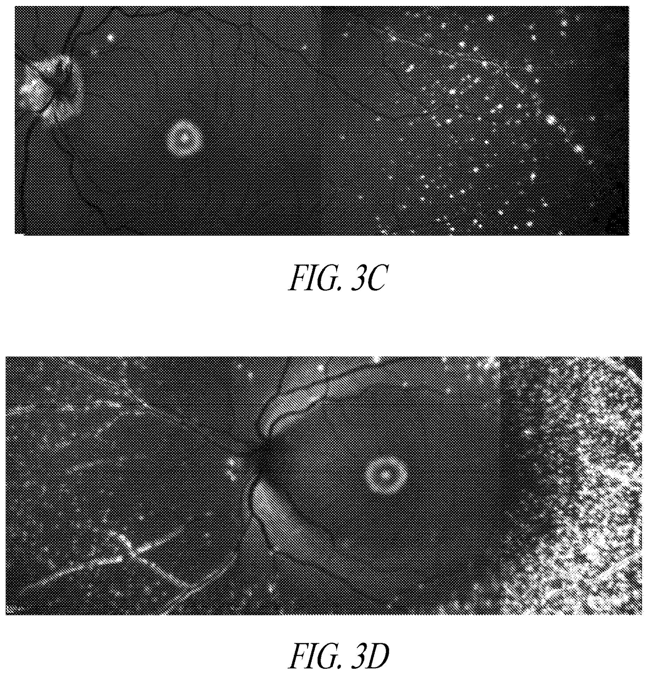

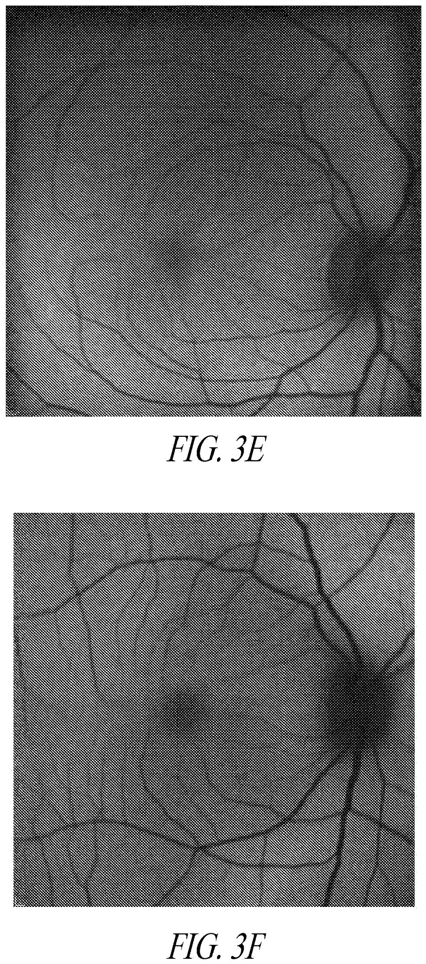

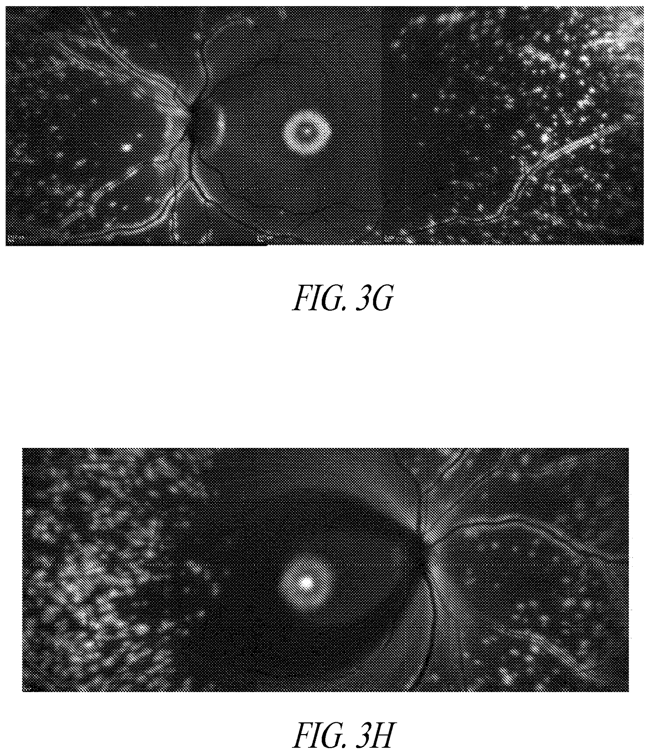

**FIGS. 3A-3H****: Heidelberg Spectralis images of AGM retina at 4 weeks.** Fluorescence imaging was performed at week 4 to assess GFP expression, as a measure of nAb evasion FIG. 3A depicts an AGM receiving an intravitreal IVIg injection (1.85 mg/mL) with a dose of 7m8.CMV-GFP of 1 × 10¹¹ vg IVT. FIG. 3B depicts an AGM receiving an intravitreal injection of mouse monoclonal α-AAV2 (5 µg/mL) with a dose of 7m8.CMV-GFP of 1 × 10¹¹ vg IVT. FIG. 3C depicts an AGM receiving an intravitreal injection of vehicle (50 µL) with a dose of 7m8.CMV-GFP of 1 × 10¹¹ vg IVT. FIG. 3D depicts an AGM receiving an intravitreal injection of IVIg (0.46 mg/mL) with a dose of 7m8.CMV-GFP of 1 × 10¹¹ vg IVT. FIG. 3E depicts an AGM receiving an intravitreal injection of IVIg (1.85 mg/mL) with a dose of 7m8.CMV-GFP of 5 × 10¹¹ vg IVT. FIG. 3F depicts an AGM receiving an intravitreal injection of mouse monoclonal α-AAV2 (5 µg/mL) with a dose of 7m8.CMV-GFP of 5 × 10¹¹ vg IVT. FIG. 3G depicts an AGM receiving an intravitreal injection of vehicle (50 µL) with a dose of 7m8.CMV-GFP of 5 × 10¹¹ vg IVT. FIG. 3H depicts an AGM receiving an intravitreal injection of IVIg (1.85 mg/mL) with a dose of 7m8.CMV-GFP of 1 × 10¹³ vg IVT.

### DETAILED DESCRIPTION OF THE INVENTION

The present disclosure provides methods of transducing ocular cells using a recombinant AAV. Also provided are methods for promoting the expression of a transgene in ocular cells, e.g., retinal cells, in an individual, e.g., for the treatment or prophylaxis of an ocular disease or disorder. These and other objects, advantages, and features of the invention will become apparent to those persons skilled in the art upon reading the details of the compositions and methods as more fully described below.

### DEFINITIONS

A "vector" as used herein refers to a macromolecule or association of macromolecules that comprises or associates with a polynucleotide and which can be used to mediate delivery of the polynucleotide to a cell. Illustrative vectors include, for example, plasmids, viral vectors, liposomes, and other gene delivery vehicles.

The term "AAV" is an abbreviation for adeno-associated virus, and may be used to refer to the virus itself or derivatives thereof. The term covers all subtypes and both naturally occurring and recombinant forms, except where required otherwise. The term "AAV" includes AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), avian AAV, bovine AAV, canine AAV, equine AAV, primate AAV, non-primate AAV, and ovine AAV. "Primate AAV" refers to AAV that infect primates, "non-primate AAV" refers to AAV that infect non-primate mammals, "bovine AAV" refers to AAV that infect bovine mammals, etc.

The genomic sequences of various serotypes of AAV, as well as the sequences of the native terminal repeats (TRs), Rep proteins, and capsid subunits are known in the art. Such sequences may be found in the literature or in public databases such as GenBank. See, e.g., GenBank Accession Numbers NC_002077 (AAV-1), AF063497 (AAV-1), NC_001401 (AAV-2), AF043303 (AAV-2), NC_001729 (AAV-3), NC_-001829 (AAV-4), U89790 (AAV-4), NC_006152 (AAV-5), AF028704 and AAB95450 (AAV-6), AF513851 (AAV-7), AF513852 (AAV-8), and NC_006261 (AAV-8), the disclosures of which are incorporated by reference herein for teaching AAV nucleic acid and amino acid sequences. See also, e.g., Srivistava et al. (1983) J. Virology 45:555; Chiorini et al. (1998) J. Virology 71:6823; Chiorini et al. (1999) J. Virology 73:1309; Bantel-Schaal et al. (1999) J. Virology 73:939; Xiao et al. (1999) J. Virology 73:3994; Muramatsu et al. (1996) Virology 221:208; Shade et al. (1986) J. Virol. 58:921; Gao et al. (2002) Proc. Nat. Acad. Sci. USA 99:11854; Moris et al. (2004) Virology 33:375-383; international patent publications WO 00/28061, WO 99/61601, WO 98/11244; and U.S. Pat. No. 6,156,303. In addition, polynucleotide sequences encoding any of the capsid proteins may be readily generated based on the amino acid sequence and the known genetic code, including codon-optimized sequences.

An "AAV virus" or "AAV viral particle" or "rAAV vector particle" or rAAV" refers to a viral particle composed of at least one AAV capsid protein (e.g., by all of the capsid proteins of a wild-type AAV) and an encapsidated polynucleotide rAAV vector. If the particle comprises a heterologous polynucleotide (i.e. a polynucleotide other than a wild-type AAV genome such as a transgene to be delivered to a mammalian cell), it is typically referred to as a "rAAV vector particle" or simply a "rAAV vector" or "rAAV". Production of rAAV particle necessarily includes production of rAAV vector, as such a vector is contained within a rAAV particle.

The term "replication defective" as used herein relative to an AAV viral vector of the invention means the AAV vector cannot independently replicate and package its genome. For example, when a cell of a subject is infected with rAAV virions, the heterologous gene is expressed in the infected cells, however, due to the fact that the infected cells lack AAV rep and cap genes and accessory function genes, the rAAV is not able to replicate further.

An "AAV variant" or "AAV mutant" as used herein refers to a viral particle composed of: a) a variant AAV capsid protein, where the variant AAV capsid protein comprises at least one amino acid difference (e.g., amino acid substitution, amino acid insertion, amino acid deletion) relative to a corresponding parental AAV capsid protein, where the AAV capsid protein does not correspond to the amino acid sequence present of a naturally occurring AAV capsid protein; and, optionally, b) a heterologous nucleic acid comprising a nucleotide sequence encoding a heterologous gene product, wherein the variant AAV capsid protein confers increased binding to heparan or a heparan sulfate proteoglycan as compared to the binding by an AAV virion comprising the corresponding parental AAV capsid protein. In certain embodiments, the variant capsid protein confers: a) increased infectivity of a retinal cell compared to the infectivity of the retinal cell by an AAV virion comprising the corresponding parental AAV capsid protein; b) altered cellular tropism as compared to the tropism of an AAV virion comprising the corresponding parental AAV capsid protein; and/or c) an increased ability to bind and/or cross the ILM as compared to an AAV virion comprising the corresponding parental AAV capsid protein.

The abbreviation "rAAV" refers to recombinant adeno-associated virus, also referred to as a recombinant AAV vector (or "rAAV vector"). A "rAAV vector" as used herein refers to an AAV vector comprising a polynucleotide sequence not of AAV origin (i.e., a polynucleotide heterologous to AAV), typically a sequence of interest for the genetic transformation of a cell. In general, the heterologous polynucleotide is flanked by at least one, and generally by two AAV inverted terminal repeat sequences (ITRs). The term rAAV vector encompasses both rAAV vector particles and rAAV vector plasmids.

"Packaging" refers to a series of intracellular events that result in the assembly and encapsidation of an AAV particle.

AAV "rep" and "cap" genes refer to polynucleotide sequences encoding replication and encapsidation proteins of adeno-associated virus. AAV rep and cap are referred to herein as AAV "packaging genes."

A "helper virus" for AAV refers to a virus that allows AAV (e.g. wild-type AAV) to be replicated and packaged by a mammalian cell. A variety of such helper viruses for AAV are known in the art, including adenoviruses, herpesviruses and poxviruses such as vaccinia. The adenoviruses encompass a number of different subgroups, although Adenovirus type 5 of subgroup C is most commonly used. Numerous adenoviruses of human, non-human mammalian and avian origin are known and available from depositories such as the ATCC. Viruses of the herpes family include, for example, herpes simplex viruses (HSV) and Epstein-Barr viruses (EBV), as well as cytomegaloviruses (CMV) and pseudorabies viruses (PRV); which are also available from depositories such as ATCC.

"Helper virus function(s)" refers to function(s) encoded in a helper virus genome which allow AAV replication and packaging (in conjunction with other requirements for replication and packaging described herein). As described herein, "helper virus function" may be provided in a number of ways, including by providing helper virus or providing, for example, polynucleotide sequences encoding the requisite function(s) to a producer cell in trans. For example, a plasmid or other expression vector comprising nucleotide sequences encoding one or more adenoviral proteins is transfected into a producer cell along with an rAAV vector.

An "infectious" virus or viral particle is one that comprises a competently assembled viral capsid and is capable of delivering a polynucleotide component into a cell for which the viral species is tropic. The term does not necessarily imply any replication capacity of the virus. Assays for counting infectious viral particles are described elsewhere in this disclosure and in the art. Viral infectivity can be expressed as the ratio of infectious viral particles to total viral particles. Methods of determining the ratio of infectious viral particle to total viral particle are known in the art. See, e.g., Grainger et al. (2005) Mol. Ther. 11:S337 (describing a TCID50 infectious titer assay); and Zolotukhin et al. (1999) Gene Ther. 6:973. See also the Examples.

A "replication-competent" virus (e.g. a replication-competent AAV) refers to a phenotypically wild-type virus that is infectious, and is also capable of being replicated in an infected cell (i.e. in the presence of a helper virus or helper virus functions). In the case of AAV, replication competence generally requires the presence of functional AAV packaging genes. In general, rAAV vectors as described herein are replication-incompetent in mammalian cells (especially in human cells) by virtue of the lack of one or more AAV packaging genes. Typically, such rAAV vectors lack any AAV packaging gene sequences in order to minimize the possibility that replication competent AAV are generated by recombination between AAV packaging genes and an incoming rAAV vector. In many embodiments, rAAV vector preparations as described herein are those which contain few if any replication competent AAV (rcAAV, also referred to as RCA) (e.g., less than about 1 rcAAV per 10.sup.2 rAAV particles, less than about 1 rcAAV per 10.sup.4 rAAV particles, less than about 1 rcAAV per 10.sup.8 rAAV particles, less than about 1 rcAAV per 10. sup. 12 rAAV particles, or no rcAAV).

The term "polynucleotide" refers to a polymeric form of nucleotides of any length, including deoxyribonucleotides or ribonucleotides, or analogs thereof. A polynucleotide may comprise modified nucleotides, such as methylated nucleotides and nucleotide analogs, and may be interrupted by non-nucleotide components. If present, modifications to the nucleotide structure may be imparted before or after assembly of the polymer. The term polynucleotide, as used herein, refers interchangeably to double- and single-stranded molecules. Unless otherwise specified or required, any embodiment of the invention described herein that is a polynucleotide encompasses both the double-stranded form and each of two complementary single-stranded forms known or predicted to make up the double-stranded form.

A polynucleotide or polypeptide has a certain percent "sequence identity" to another polynucleotide or polypeptide, meaning that, when aligned, that percentage of bases or amino acids are the same when comparing the two sequences. Sequence similarity can be determined in a number of different manners. To determine sequence identity, sequences can be aligned using the methods and computer programs, including BLAST, available over the world wide web at ncbi.nlm.nih.gov/BLAST/. Another alignment algorithm is FASTA, available in the Genetics Computing Group (GCG) package, from Madison, Wis., USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, Calif., USA. Of particular interest are alignment programs that permit gaps in the sequence. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. See J. Mol. Biol. 48: 443-453 (1970)

Of interest is the BestFit program using the local homology algorithm of Smith and Waterman (Advances in Applied Mathematics 2: 482-489 (1981) to determine sequence identity. The gap generation penalty will generally range from 1 to 5, usually 2 to 4 and in many embodiments will be 3. The gap extension penalty will generally range from about 0.01 to 0.20 and in many instances will be 0.10. The program has default parameters determined by the sequences inputted to be compared. Preferably, the sequence identity is determined using the default parameters determined by the program. This program is available also from Genetics Computing Group (GCG) package, from Madison, Wis., USA.

Another program of interest is the FastDB algorithm. FastDB is described in Current Methods in Sequence Comparison and Analysis, Macromolecule Sequencing and Synthesis, Selected Methods and Applications, pp. 127-149, 1988, Alan R. Liss, Inc. Percent sequence identity is calculated by FastDB based upon the following parameters: Mismatch Penalty: 1.00; Gap Penalty: 1.00; Gap Size Penalty: 0.33; and Joining Penalty: 30.0.

A "gene" refers to a polynucleotide containing at least one open reading frame that is capable of encoding a particular gene product after being transcribed, and sometimes also translated. The term "gene" or "coding sequence" refers to a nucleotide sequence in vitro or in vivo that encodes a gene product. In some instances, the gene consists or consists essentially of coding sequence, that is, sequence that encodes the gene product. In other instances, the gene comprises additional, non-coding, sequence. For example, the gene may or may not include regions preceding and following the coding region, e.g. 5' untranslated (5' UTR) or "leader" sequences and 3' UTR or "trailer" sequences, as well as intervening sequences (introns) between individual coding segments (exons).

A "gene product" is a molecule resulting from expression of a particular gene. Gene products include, e.g., a polypeptide, an aptamer, an interfering RNA, an mRNA, and the like. In particular embodiments, a "gene product" is a polypeptide, peptide, protein or interfering RNA including short interfering RNA (siRNA), miRNA or small hairpin RNA (shRNA). In particular embodiments, a gene product is a therapeutic gene product, e.g., a therapeutic protein.

As used herein, a "therapeutic gene" refers to a gene that, when expressed, produces a therapeutic gene product that confers a beneficial effect on the cell or tissue in which it is present, or on a mammal in which the gene is expressed. Examples of beneficial effects include amelioration of a sign or symptom of a condition or disease, prevention or inhibition of a condition or disease, or conferral of a desired characteristic. Therapeutic genes include, but are not limited to, genes that correct a genetic deficiency in a cell or mammal.

As used herein, a "transgene" is a gene that is delivered to a cell by a vector. The gene may comprise or consist of a sequence that encodes a gene product.

"Recombinant," as applied to a polynucleotide means that the polynucleotide is the product of various combinations of cloning, restriction or ligation steps, and other procedures that result in a construct that is distinct from a polynucleotide found in nature. A recombinant virus is a viral particle comprising a recombinant polynucleotide. The terms respectively include replicates of the original polynucleotide construct and progeny of the original virus construct.

A "control element" or "control sequence" is a nucleotide sequence involved in an interaction of molecules that contributes to the functional regulation of a polynucleotide, including replication, duplication, transcription, splicing, translation, or degradation of the polynucleotide. The regulation may affect the frequency, speed, or specificity of the process, and may be enhancing or inhibitory in nature. Control elements known in the art include, for example, transcriptional regulatory sequences such as promoters and enhancers. A promoter is a DNA region capable under certain conditions of binding RNA polymerase and initiating transcription of a coding region usually located downstream (in the 3' direction) from the promoter.

"Operatively linked" or "operably linked" refers to a juxtaposition of genetic elements, wherein the elements are in a relationship permitting them to operate in the expected manner. For instance, a promoter is operatively linked to a coding region if the promoter helps initiate transcription of the coding sequence. There may be intervening residues between the promoter and coding region so long as this functional relationship is maintained.

An "expression vector" is a vector comprising a region which encodes a gene product of interest, and is used for effecting the expression of the gene product in an intended target cell. An expression vector also comprises control elements operatively linked to the encoding region to facilitate expression of the gene product in the target. The combination of control elements and a gene or genes to which they are operably linked for expression is sometimes referred to as an "expression cassette," a large number of which are known and available in the art or can be readily constructed from components that are available in the art.

"Heterologous" means derived from a genotypically distinct entity from that of the rest of the entity to which it is being compared. For example, a polynucleotide introduced by genetic engineering techniques into a plasmid or vector derived from a different species is a heterologous polynucleotide. A promoter removed from its native coding sequence and operatively linked to a coding sequence with which it is not naturally found linked is a heterologous promoter. Thus, for example, an rAAV that includes a heterologous nucleic acid encoding a heterologous gene product is an rAAV that includes a nucleic acid not normally included in a naturally-occurring, wild-type AAV, and the encoded heterologous gene product is a gene product not normally encoded by a naturally-occurring, wild-type AAV.

As used herein, the terms "polypeptide," "peptide," and "protein" refer to polymers of amino acids of any length. The terms also encompass an amino acid polymer that has been modified; for example, disulfide bond formation, glycosylation, lipidation, phosphorylation, or conjugation with a labeling component.

By "comprising" it is meant that the recited elements are required in, for example, the composition, method, kit, etc., but other elements may be included to form the, for example, composition, method, kit etc. within the scope of the claim. For example, an expression cassette "comprising" a gene encoding a therapeutic polypeptide operably linked to a promoter is an expression cassette that may include other elements in addition to the gene and promoter, e.g. poly-adenylation sequence, enhancer elements, other genes, linker domains, etc.

By "consisting essentially of', it is meant a limitation of the scope of the, for example, composition, method, kit, etc., described to the specified materials or steps that do not materially affect the basic and novel characteristic(s) of the, for example, composition, method, kit, etc. For example, an expression cassette "consisting essentially of' a gene encoding a therapeutic polypeptide operably linked to a promoter and a polyadenylation sequence may include additional sequences, e.g. linker sequences, so long as they do not materially affect the transcription or translation of the gene. As another example, a variant, or mutant, polypeptide fragment "consisting essentially of' a recited sequence has the amino acid sequence of the recited sequence plus or minus about 10 amino acid residues at the boundaries of the sequence based upon the full length naive polypeptide from which it was derived, e.g. 10, 9, 8, 7, 6, 5, 4, 3, 2 or 1 residue less than the recited bounding amino acid residue, or 1, 2, 3, 4, 5, 6, 7, 8, 9, or 10 residues more than the recited bounding amino acid residue.

By "consisting of', it is meant the exclusion from the composition, method, or kit of any element, step, or ingredient not specified in the claim. For example, an expression cassette "consisting of' a gene encoding a therapeutic polypeptide operably linked to a promoter, and a polyadenylation sequence consists only of the promoter, polynucleotide sequence encoding the therapeutic polypeptide, and polyadenlyation sequence. As another example, a polypeptide "consisting of' a recited sequence contains only the recited sequence.

A "promoter" as used herein encompasses a DNA sequence that directs the binding of RNA polymerase and thereby promotes RNA synthesis, i.e., a minimal sequence sufficient to direct transcription. Promoters and corresponding protein or polypeptide expression may be ubiquitous, meaning strongly active in a wide range of cells, tissues and species or cell-type specific, tissue-specific, or species specific. Promoters may be "constitutive," meaning continually active, or "inducible," meaning the promoter can be activated or deactivated by the presence or absence of biotic or abiotic factors. Also included in the nucleic acid constructs or vectors of the invention are enhancer sequences that may or may not be contiguous with the promoter sequence. Enhancer sequences influence promoter-dependent gene expression and may be located in the 5' or 3' regions of the native gene.

An "enhancer" as used herein encompasses a cis-acting element that stimulates or inhibits transcription of adjacent genes. An enhancer that inhibits transcription also is termed a "silencer". Enhancers can function (i.e., can be associated with a coding sequence) in either orientation, over distances of up to several kilobase pairs (kb) from the coding sequence and from a position downstream of a transcribed region.

A "termination signal sequence" as used herein encompasses any genetic element that causes RNA polymerase to terminate transcription, such as for example a polyadenylation signal sequence.

A "polyadenylation signal sequence" as used herein encompasses a recognition region necessary for endonuclease cleavage of an RNA transcript that is followed by the polyadenylation consensus sequence AATAAA. A polyadenylation signal sequence provides a "polyA site", i.e. a site on a RNA transcript to which adenine residues will be added by post-transcriptional polyadenylation.

The term "endogenous" as used herein with reference to a nucleotide molecule or gene product refers to a nucleic acid sequence, e.g. gene or genetic element, or gene product, e.g. RNA, protein, that is naturally occurring in or associated with a host virus or cell.

The term "native" as used herein refers to a nucleotide sequence, e.g. gene, or gene product, e.g. RNA, protein, that is present in a wildtype virus or cell. The term "variant" as used herein refers to a mutant of a reference polynucleotide or polypeptide sequence, for example a native polynucleotide or polypeptide sequence, i.e. having less than 100% sequence identity with the reference polynucleotide or polypeptide sequence. Put another way, a variant comprises at least one amino acid difference (e.g., amino acid substitution, amino acid insertion, amino acid deletion) relative to a reference polynucleotide sequence, e.g. a native polynucleotide or polypeptide sequence. For example, a variant may be a polynucleotide having a sequence identity of 70% or more with a full length native polynucleotide sequence, e.g. an identity of 75% or 80% or more, such as 85%, 90%, or 95% or more, for example, 98% or 99% identity with the full length native polynucleotide sequence. As another example, a variant may be a polypeptide having a sequence identity of 70% or more with a full length native polypeptide sequence, e.g. an identity of 75% or 80% or more, such as 85%, 90%, or 95% or more, for example, 98% or 99% identity with the full length native polypeptide sequence. Variants may also include variant fragments of a reference, e.g. native, sequence sharing a sequence identity of 70% or more with a fragment of the reference, e.g. native, sequence, e.g. an identity of 75% or 80% or more, such as 85%, 90%, or 95% or more, for example, 98% or 99% identity with the native sequence.

As used herein, the terms "biological activity" and "biologically active" refer to the activity attributed to a particular biological element in a cell. For example, the "biological activity" of an "immunoglobulin", "antibody" or fragment or variant thereof refers to the ability to bind an antigenic determinant and thereby facilitate immunological function. As another example, the biological activity of a polypeptide or functional fragment or variant thereof refers to the ability of the polypeptide or functional fragment or variant thereof to carry out its native functions of, e.g., binding, enzymatic activity, etc. As a third example, the biological activity of a gene regulatory element, e.g. promoter, enhancer, kozak sequence, and the like, refers to the ability of the regulatory element or functional fragment or variant thereof to regulate, i.e. promote, enhance, or activate the translation of, respectively, the expression of the gene to which it is operably linked.

The terms "administering" or "introducing", as used herein, refer to delivery of a vector for recombinant gene or protein expression to a cell, to cells and/or organs of a subject, or to a subject. Such administering or introducing may take place in vivo, in vitro or ex vivo. A vector for expression of a gene product may be introduced into a cell by transfection, which typically means insertion of heterologous DNA into a cell by physical means (e.g., calcium phosphate transfection, electroporation, microinjection or lipofection); infection, which typically refers to introduction by way of an infectious agent, i.e. a virus; or transduction, which typically means stable infection of a cell with a virus or the transfer of genetic material from one microorganism to another by way of a viral agent (e.g., a bacteriophage).

Typically, a cell is referred to as "transduced", "infected"; "transfected" or "transformed" dependent on the means used for administration, introduction or insertion of heterologous DNA (i.e., the vector) into the cell. The terms "transduced", "transfected" and "transformed" may be used interchangeably herein regardless of the method of introduction of heterologous DNA.

The term "host cell", as used herein refers to a cell which has been transduced, infected, transfected or transformed with a vector. The vector may be a plasmid, a viral particle, a phage, etc. The culture conditions, such as temperature, pH and the like, are those previously used with the host cell selected for expression, and will be apparent to those skilled in the art. It will be appreciated that the term "host cell" refers to the original transduced, infected, transfected or transformed cell and progeny thereof.

The terms "treatment", "treating" and the like are used herein to generally mean obtaining a desired pharmacologic and/or physiologic effect. The effect may be prophylactic in terms of completely or partially preventing a disease or symptom thereof, e.g. reducing the likelihood that the disease or symptom thereof occurs in the subject, and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease. The therapeutic agent may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease.

The terms "individual," "host," "subject," and "patient" are used interchangeably herein, and refer to a mammal, including, but not limited to, human and non-human primates, including simians and humans; mammalian sport animals (e.g., horses); mammalian farm animals (e.g., sheep, goats, etc.); mammalian pets (dogs, cats, etc.); and rodents (e.g., mice, rats, etc.).

By "neutralizing antibody" or "nAb" is meant an antibody which recognizes a specific antigen and inhibits the effect(s) of the antigen in the host (e. g., a human). As used herein, the antibody can be a single antibody or a plurality of antibodies. In certain embodiments, a neutralizing antibody can inhibit the effect(s) of the antigen of at least 1, 2, 3, 4, 5, 6, 7, 8, 9 or more difference strains of AAV. For example, the neutralizing antibody can inhibit infectivity of (e.g., cell entry), or gene expression directed by, an AAV. Neutralizing antibodies that bind a virus, e.g., an AAV can, for example, exert a substantial deleterious effect on infectivity by, transduction by, gene expression directed by, or another property or activity of the virus.

The various compositions and methods of the invention are described below. Although particular compositions and methods are exemplified herein, it is understood that any of a number of alternative compositions and methods are applicable and suitable for use in practicing the invention. It will also be understood that an evaluation of the expression constructs and methods of the invention may be carried out using procedures standard in the art.

The practice of the present invention will employ, unless otherwise indicated, conventional techniques of cell biology, molecular biology (including recombinant techniques), microbiology, biochemistry and immunology, which are within the scope of those of skill in the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); and "Current Protocols in Immunology" (J. E. Coligan et al., eds., 1991), each of which is expressly incorporated by reference herein.

Several aspects of the invention are described below with reference to example applications for illustration. It should be understood that numerous specific details, relationships, and methods are set forth to provide a full understanding of the invention. One having ordinary skill in the relevant art, however, will readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

All publications mentioned herein are incorporated herein by reference to disclose and describe the methods and/or materials in connection with which the publications are cited. It is understood that the present disclosure supersedes any disclosure of an incorporated publication to the extent there is a contradiction.

It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Nothing-herein is to be construed as an admission that the present invention is not entitled to antedate such publication by virtue of prior invention. Further, the dates of publication provided may be different from the actual publication dates which may need to be independently confirmed.

Unless otherwise indicated, all terms used herein have the same meaning as they would to one skilled in the art and the practice of the present invention will employ, conventional techniques of microbiology and recombinant DNA technology, which are within the knowledge of those of skill of the art.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. For example, while certain methods disclosed herein are exemplified by referring to rAAV, it is understood that any of these methods may be practiced using a different viral vector, including but not limited to those specifically disclosed herein. It is intended that the following claims define the scope of the invention and that methods and structures within the scope of these claims and their equivalents be covered thereby.

### VIRUS

In particular embodiments, methods disclosed herein are practiced using a recombinant virus or virion comprising a transgene that encodes a gene product. The recombinant virus may be used to transduce a cell, wherein the transgene expresses the gene product, in order to deliver the gene product to the cell or a subject comprising the cell.

In certain embodiments, the virus or virion is a viral vector derived from a virus, e.g., an adenovirus, an adeno-associated virus (AAV), a lentivirus, a herpes virus, an alpha virus or a retrovirus, e.g., Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) or lentivirus. While embodiments encompassing the use of adeno-associated virus are described in greater detail herein, it is expected that the ordinarily skilled artisan will appreciate that similar knowledge and skill in the art can be brought to bear on non-AAV gene therapy vectors as well. See, for example, the discussion of retroviral vectors in, e.g., US Patent No. 7,585,676 and US Patent No. 8,900,858, and the discussion of adenoviral vectors in, e.g. US Patent No. 7,858,367, the full disclosures of which are incorporated herein by reference. In certain embodiments, the recombinant virus or virion is infectious. In certain embodiments, the recombinant virion or virus is replication-competent. In certain embodiments, the recombinant virus or virion is replication-incompetent.

In particular embodiments, the virus is an adeno-associated virus (rAAV). In particular embodiments, the virus is an AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), avian AAV, bovine AAV, canine AAV, equine AAV, primate AAV, non-primate AAV, or ovine AAV

In certain embodiment, the virus is a variant AAV. In particular embodiments, the AAV comprises a variant AAV capsid protein, e.g., the AAV capsid protein may comprises an insertion within a capsid protein, e.g., VP1, of AAV type 1 (AAV-1), AAV type 2 (AAV-2), AAV type 3 (AAV-3), AAV type 4 (AAV-4), AAV type 5 (AAV-5), AAV type 6 (AAV-6), AAV type 7 (AAV-7), AAV type 8 (AAV-8), AAV type 9 (AAV-9), AAV type 10 (AAV-10), AAV rh.10, avian AAV, bovine AAV, canine AAV, equine AAV, primate AAV, non-primate AAV, bovine AAV, AAV2/7m8, AAVShH10, AAV2.5T, AAV2.5T/7m8, AAV9/7m8, and AAV5/7m8. AAV2.5T capsid proteins and virions are described in U.S. Patent No. 9,233,131, in which the VP1-encoding amino acid sequences of AAV2.5T is provided as SEQ ID NO:42 and Figures 10A-B. AAV7m8 capsid proteins are described in U.S. Patent No. 9,193,956. AAV7m8 includes a 7m8 insert between amino acids 587 and 588 of the wildtype AAV2 genome. AAV2.5T/7m8 capsid proteins correspond to AAV2.5T capsid proteins further comprising a 7m8 insert.

In particular embodiments, the virions and viral vectors having one or more modified or altered capsid protein exhibit: 1) increased infectivity of a retinal cell; 2) increased tissue specificity for a retinal cell as compared to one or more other cells or tissues; 3) increased binding to heparan or heparan sulfate proteoglycans and/or the inner limiting membrane (ILM); 4) an increased ability to infect and/or deliver a gene product across the ILM when administered intravitreally, as compared to a corresponding virion comprising its native or wild-type capsid protein instead of the modified capsid protein. In some embodiment, the retinal cell is a photoreceptor cell (e.g., rods or cones). In other cases, the retinal cell is an RGC. In other cases, the retinal cell is an RPE cell. In other cases, the retinal cell is a Muller cell. In other embodiments, the retinal cell is an amacrine cell, bipolar cell, or horizontal cells.

In certain embodiments, the variant capsid protein, e.g., VP1, includes an insertion of a peptide of from about 5 amino acids to about 11 amino acids in length. In particular embodiments, the insertion peptide has a length of 5 amino acids, 6 amino acids, 7 amino acids, 8 amino acids, 9 amino acids, 10 amino acids, or 11 amino acids. These insertions are collectively referred to as "7m8 insertions."

In certain embodiments, the variant capsid protein comprises a "7m8 insertion." In certain embodiments, the 7m8 insertion peptide comprises an amino acid sequence of any one of the formulas set forth herein. For example, a 7m8 insertion peptide can be a peptide of from 5 to 11 amino acids in length, where the insertion peptide is of Formula I:

Y₁Y₂X₁X₂X₃X₄X_{S}X₆X₇Y₃Y₄

where:
each of Y₁-Y₄ is independently absent or present and, if present, is independently selected from Ala, Leu, Gly, Ser, and Thr;
X₁ is absent or present and, if present, is selected from Leu, Asn, and Lys;
X₂ is selected from Gly, Glu, Ala, and Asp;
X₃ is selected from Glu, Thr, Gly, and Pro;
X₄ is selected from Thr, Ile, Gln, and Lys;
X₅ is selected from Thr and Ala;
X₆ is selected from Arg, Asn, and Thr; and
X₇ is absent or present and, if present, is selected from Pro and Asn.

As another example, a 7m8 insertion peptide can be a peptide of from 5 to 11 amino acids in length, where the insertion peptide is of Formula IIa:

Y₁Y₂X₁X₂X₃X₄X_{S}X₆X₇Y₃Y₄

where:
each of Y₁-Y₄ are independently absent or present and, if present, is independently selected from Ala, Leu, Gly, Ser, and Thr;
each of X₁-X₄ is any amino acid;
X₅ is Thr;
X₆ is Arg; and
and X₇ is Pro.

As another example, a 7m8 insertion peptide can be a peptide of from 5 to 11 amino acids in length, where the insertion peptide is of Formula IIb:

Y₁Y₂X₁X₂X₃X₄X₅X₆X₇Y₃Y₄

where:
each of Y₁-Y₄ is independently absent or present and, if present, is independently selected from Ala, Leu, Gly, Ser, and Thr;
X₁ is absent or present and, if present, is selected from Leu and Asn;
X₂ is absent or present and, if present, is selected from Gly and Glu;
X₃ is selected from Glu and Thr;
X₄ is selected from Thr and Ile;
X₅ is Thr;
X₆ is Arg; and
X₇ is Pro.

As another example, a 7m8 insertion peptide can be a peptide of from 5 to 11 amino acids in length, where the insertion peptide is of Formula III:

Y₁Y₂X₁X₂X₃X₄X_{S}X₆X₇Y₃Y₄

where:
each of Y₁-Y₄ is independent absent or present and, if present, is independently selected from Ala, Leu, Gly, Ser, and Thr;
X₁ is absent or present and, if present, is Lys;
X₂ is selected from Ala and Asp;
X₃ is selected from Gly and Pro;
X₄ is selected from Gln and Lys;
X₅ is selected from Thr and Ala;
X₆ is selected from Asn and Thr; and
X₇ is absent or present and, if present, is Asn.

As another example, a 7m8 insertion peptide can be a peptide of from 5 to 11 amino acids in length, where the insertion peptide is of Formula IV:

Y₁Y₂X₁X₂X₃X₄X₅X₆X₇Y₃Y₄

where:
each of Y₁-Y₄ is independently absent or present and, if present, is independently selected from Ala, Leu, Gly, Ser, and Thr;
X₁ is absent or present, if present, is a positively charged amino acid or an uncharged amino acid; or is selected from Leu, Asn, Arg, Ala, Ser, and Lys;
X₂ is a negatively charged amino acid or an uncharged amino acid; or is selected from Gly, Glu, Ala, Val, Thr, and Asp;
X₃ is a negatively charged amino acid or an uncharged amino acid; or is selected from Glu, Thr, Gly, Asp, or Pro;
X₄ is selected from Thr, Ile, Gly, Lys, Asp, and Gln;
X₅ is a polar amino acid, an alcohol (an amino acid having a free hydroxyl group), or a hydrophobic amino acid; or is selected from Thr, Ser, Val, and Ala;
X₆ is a positively charged amino acid or an uncharged amino acid; or is selected from Arg, Val, Lys, Pro, Thr, and Asn; and
X₇ is absent or present and, if present, is a positively charged amino acid or an uncharged amino acid; or is selected from Pro, Gly, Phe, Asn, and Arg.

As non-limiting examples, the 7m8 insertion peptide can comprise or consist of an amino acid sequence selected from LGETTRP, NETITRP, KAGQANN, KDPKTTN, KDTDTTR, RAGGSVG, AVDTTKF, and STGKVPN.

In some cases, the 7m8 insertion peptide has from 1 to 4 spacer amino acids (Y₁-Y4) at the amino terminus and/or at the carboxyl terminus of any one of LGETTRP, NETITRP, KAGQANN, KDPKTTN, KDTDTTR, RAGGSVG, AVDTTKF, and STGKVPN. Suitable spacer amino acids include, but are not limited to, leucine, alanine, glycine, and serine.

For example, in some cases, a 7m8 insertion peptide has one of the following amino acid sequences: LALGETTRPA; LANETITRPA, LAKAGQANNA, LAKDPKTTNA, LAKDTDTTRA, LARAGGSVGA, LAAVDTTKFA, and LASTGKVPNA. As another example, in some cases, a 7m8 insertion peptide has one of the following amino acid sequences: AALGETTRPA; AANETITRPA, AAKAGQANNA, and AAKDPKTTNA. As yet another example, in some cases, a 7m8 insertion peptide has one of the following amino acid sequences: GLGETTRPA; GNETITRPA, GKAGQANNA, and GKDPKTTNA. As another example, in some cases, an insertion peptide comprises one of KDTDTTR, RAGGSVG, AVDTTKF, and STGKVPN, flanked on the C-terminus by AA and on the N-terminus by A; or comprises one of KDTDTTR, RAGGSVG), AVDTTKF, and STGKVPN flanked on the C-terminus by G and on the N-terminus by A. In certain embodiments, the 7m8 is a random sequence of five to 12 amino acid residues.

In certain embodiment, the 7m8 amino acid insert comprises or consists of the following amino acid sequence: LGETTRP. In particular embodiments, the 7m8 insert comprises or consists of the amino acid sequence: LALGETTRPA, or a fragment comprising at least five, at least six, at least seven, at least eight, or at least nine consecutive amino acids thereof. In particular embodiments, the 7m8 insert comprises or consists of an amino acid sequence having at least 80%, at least 85%, or at least 90% homology to the amino acid sequence: LALGETTRPA, or a fragment comprising at least five, at least six, at least seven, at least eight, or at least nine consecutive amino acids thereof. In some embodiments, a capsid protein includes an m78 insertion comprising an amino acid sequence having at least about 85%, at least about 90%, at least about 95%, at least about 98%, at least about 99%, or 100%, amino acid sequence identity to an amino acid sequence selected from LGETTRP and LALGETTRPA.

In some embodiments, a variant AAV capsid polypeptide is a chimeric capsid, e.g., the capsid comprises a portion of an AAV capsid of a first AAV serotype and a portion of an AAV capsid of a second serotype; and in some embodiments, it further comprises a 7m8 insertion relative to a corresponding parental AAV capsid protein.

In some embodiments, a subject variant capsid protein comprises an amino acid sequence having at least about 85%, at least about 90%, at least about 95%, at least about 98%, or at least about 99%, amino acid sequence identity to a wild type or parental capsid protein, e.g., VP1, and a 7m8 insertion relative to the corresponding wild type or parental AAV capsid protein.

In some embodiments, the recombinant virion or virus, e.g., an AAV, comprises a polynucleotide cassette comprising a transgene sequence that encodes a gene product, e.g., a therapeutic gene product. In certain embodiments, the transgene sequence that encodes the gene product is operably linked to a promoter sequence. In certain embodiments, the polynucleotide cassette is flanked on the 5' and 3' ends by functional AAV inverted terminal repeat (ITR) sequences. By "functional AAV ITR sequences" is meant that the ITR sequences function as intended for the rescue, replication and packaging of the AAV virion. Hence, AAV ITRs for use in the viral vectors of the invention need not have a wild-type nucleotide sequence, and may be altered by the insertion, deletion or substitution of nucleotides or the AAV ITRs may be derived from any of several AAV serotypes, e.g. AAV1, AAV2, AAV3, AAV4, AAV5, AAV6, AAV7, AAV8, AAV9, AAV10. Certain AAV vectors have the wild type REP and CAP genes deleted in whole or part, but retain functional flanking ITR sequences.

In certain embodiment, recombinant viruses or virions described herein comprises a heterologous nucleic acid comprising a nucleotide sequence (i.e., a transgene) encoding a gene product, e.g., a therapeutic gene product. In some embodiments, the gene product is an interfering RNA. In some embodiments, the gene product is an aptamer. In some embodiments, the gene product is a polypeptide. In some embodiments, the gene product is a site-specific nuclease that provides for site-specific knock-down of gene function.

Where the gene product is an interfering RNA (RNAi), suitable RNAi include but are not limited to RNAi that decrease the level of an apoptotic or angiogenic factor in a cell. For example, an RNAi can be an shRNA or siRNA that reduces the level of a gene product that induces or promotes apoptosis in a cell. Genes whose gene products induce or promote apoptosis are referred to herein as "pro-apoptotic genes" and the products of those genes (mRNA; protein) are referred to as "pro-apoptotic gene products." Pro-apoptotic gene products include, e.g., Bax, Bid, Bak, and Bad gene products. See, e.g., U.S. Pat. No. 7,846,730.

Interfering RNAs could also be against an angiogenic product, for example VEGF (e.g., Cand5; see, e.g., U.S. Patent Publication No. 2011/0143400; U.S. Patent Publication No. 2008/0188437; and Reich et al. (2003) Mol. Vis. 9:210), VEGFR1 (e.g., Sirna-027; see, e.g., Kaiser et al. (2010) Am. J. Ophthalmol. 150:33; and Shen et al. (2006) Gene Ther. 13:225), or VEGFR2 (Kou et al. (2005) Biochem. 44:15064). See also, U.S. Pat. Nos. 6,649,596, 6,399,586, 5,661,135, 5,639,872, and 5,639,736; and U.S. Pat. Nos. 7,947,659 and 7,919,473.

Where the gene product is an aptamer, exemplary aptamers of interest include an aptamer against vascular endothelial growth factor (VEGF). See, e.g., Ng et al. (2006) Nat. Rev. Drug Discovery 5:123; and Lee et al. (2005) Proc. Natl. Acad. Sci. USA 102:18902. For example, a VEGF aptamer can comprise the nucleotide sequence 5'-cgcaaucagugaaugcuuauacauccg-3' (SEQ ID NO:17). Also suitable for use is a PDGF-specific aptamer, e.g., E10030; see, e.g., Ni and Hui (2009) Ophthalmologica 223:401; and Akiyama et al. (2006) J. Cell Physiol. 207:407).

Where the gene product is a polypeptide, in certain embodiments, the polypeptide may enhance function of a retinal cell, e.g., the function of a rod or cone photoreceptor cell, a retinal ganglion cell, a Muller cell, a bipolar cell, an amacrine cell, a horizontal cell, or a retinal pigmented epithelial cell. Illustrative polypeptides include neuroprotective polypeptides (e.g., GDNF, CNTF, NT4, NGF, and NTN); anti-angiogenic polypeptides (e.g., a soluble vascular endothelial growth factor (VEGF) receptor; a VEGF-binding antibody; a VEGF-binding antibody fragment (e.g., a single chain anti-VEGF antibody); endostatin; tumstatin; angiostatin; a soluble Flt polypeptide (Lai et al. (2005) Mol. Ther. 12:659); an Fc fusion protein comprising a soluble Flt polypeptide (see, e.g., Pechan et al. (2009) Gene Ther. 16:10); pigment epithelium-derived factor (PEDF); a soluble Tie-2 receptor; etc.); tissue inhibitor of metalloproteinases-3 (TIMP-3); a light-responsive opsin, e.g., a rhodopsin; anti-apoptotic polypeptides (e.g., Bcl-2, Bcl-Xl); and the like. Suitable polypeptides include, but are not limited to, glial derived neurotrophic factor (GDNF); fibroblast growth factor 2; neurturin (NTN); ciliary neurotrophic factor (CNTF); nerve growth factor (NGF); neurotrophin-4 (NT4); brain derived neurotrophic factor (BDNF); epidermal growth factor; rhodopsin; X-linked inhibitor of apoptosis; and Sonic hedgehog, as well as functional variants and fragments of any of these, including variants having at least 80%, at least 85%, at least 90%, or at least 95% sequence identity to any of these polypeptides, and fragments comprising at least 20%, at least 30%, at least 50%, at least 60%, at least 70%, at least 80%, or at least 90% of any of these polypeptides or variants thereof.

Suitable light-responsive opsins include, e.g., a light-responsive opsin as described in U.S. Patent Publication No. 2007/0261127 (e.g., ChR2; Chop2); U.S. Patent Publication No. 2001/0086421; U.S. Patent Publication No. 2010/0015095; and Diester et al. (2011) Nat. Neurosci. 14:387.

In certain embodiments, the gene product is an anti-angiogenic polypeptide, a vascular endothelial growth factor (VEGF)-binding protein, an anti-VEGF agent, or an opsin protein.

Vascular endothelial growth factor (herein referred to as "VEGF" or "VEGF ligand") is a potent endothelial cell-specific mitogen that plays a key role in physiological blood vessel formation. In some cases, VEGF activity results from the binding of VEGF ligand to one or more VEGF receptors in a cell. The binding of VEGF ligand to VEGF receptor may have numerous downstream cellular and biochemical effects, including but not limited to angiogenesis in tissues. VEGF has been implicated in virtually every type of angiogenic or neovascular disorder, including those associated with cancer, ischemia, and inflammation. Additionally, VEGF has been implicated in eye diseases, including but not limited to ischemic retinopathy, intraocular neovascularization, age-related macular degeneration (AMD), wet-AMD, dry-AMD, retinal neovascularization, diabetic macular edema, diabetic retina ischemia, diabetic retinal edema, proliferative diabetic retinopathy, retinal vein occlusion, central retinal vein occlusion, branched retinal vein occlusion. Further, anti-VEGF treatments, including the compositions and methods of this disclosure as described herein, may be used in the treatment of one or more of these diseases described herein. Recent data suggests that VEGF is the principal angiogenic growth factor in the pathogenesis of the wet form of AMD

VEGF, a 46-kDa homodimeric glycopeptide, is expressed by several different ocular cell types including but not limited to pigment epithelial cells, pericytes, vascular endothelial cells, neuroglia and ganglion cells. In some cases, VEGF is expressed in specific spatial and temporal patterns during retinal development. In some cases, the human isoforms of VEGF may include proteins of 206, 189, 183, 165, 148, 145, and 121 amino acids per monomer, however the predominant human VEGF isoforms include but are not limited to VEGF121, VEGF 165, VEGF 189 and VEGF206. These proteins are produced by alternative splicing of the VEGF mRNA and differ in their ability to bind to heparin and to the specific VEGF receptors or coreceptors (neuropilins). The domain encoded by exons 1-5 of the VEGF gene contains information required for the recognition of the known VEGF receptors KDR/FLK-1 and FLT-1. This domain is present in all of the VEGF isoforms. VEGF acts via these receptors, which are high- affinity receptor tyrosine kinases, leading to endothelial cell proliferation, migration, and increased vasopermeability.

VEGF is one of the several factors involved in the complex process of angiogenesis and has a very high specificity for vascular endothelial cells. VEGF is a regulator of physiological angiogenesis during processes such as embryo genesis, skeletal growth and reproductive function, but it has also been implicated in pathological angiogenesis associated with disease such as in cancer, placental disorders and other conditions. The potential biological effects of VEGF may be mediated by specific fms- like membrane spanning receptors, FLT-1 and FLK-1/KDR. In some cases, these naturally occurring binding partners of VEGF may affect binding of VEGF to VEGF receptors, thus modulating activation of the VEGF receptor and subsequent downstream pathways.

As related to cancer, several VEGF inhibitors, including a humanized monoclonal antibody to VEGF (rhuMab VEGF), an anti-VEGFR-2 antibody, small molecules inhibiting VEGFR-2 signal transduction and a soluble VEGF receptor have shown some therapeutic properties.

As related to intraocular neovascular diseases, such as diabetic retinopathy, retinal vein occlusions, or age related macular degeneration, some VEGF antagonists have shown therapeutic effects, despite the need for frequent administration.

In certain embodiments, methods disclosed herein are used to deliver an anti-VEGF agent to the eye. In particular embodiments, the recombinant virus of the present disclosure comprises a sequence encoding an anti-VEGF protein, including, but not limited to the VEGF-binding proteins or functional fragments thereof disclosed in U.S. Pat. Nos 5,712,380, 5,861,484 and 7,071,159 and VEGF-binding fusion proteins disclosed in U.S. Pat. No. 7,635,474. In one embodiment, an anti-VEGF agent is aflibercept or a variant or functional fragment thereof. An anti-VEGF protein may also include the sFLT-1 protein as described herein.

The recombinant viruses or plasmids of the present disclosure may comprise the sequence encoding an anti-VEGF protein, including the naturally occurring protein sFlt-1, as described in US Patent 5,861,484 and that sequence described by SEQ ID NO: 109. It also includes, but is not limited to functional fragments thereof, including sequences of sFlt-1 domain 2 or those set forth in SEQ ID NO: 121, as well as related constructs, such as the VEGF-binding fusion proteins disclosed in U.S. Pat. No. 7,635,474. An anti- VEGF protein may also include the sFLT-1 protein as described herein. These sequences can be expressed from DNA encoding such sequences using the genetic code, a standard technique that is understood by those skilled in the art. As can be appreciated by those with skill in the art, due to the degeneracy of the genetic code, anti-VEGF protein sequences can be readily expressed from a number of different DNA sequences.

"sFlt-1 protein" herein refers to a polypeptide sequence, or functional fragment thereof, with at least 90%, or more, homology to the naturally occurring human sFLT-1 sequence, such that the sFlt-1 protein or polypeptide binds to VEGF and/or the VEGF receptor. Homology refers to the % conservation of residues of an alignment between two sequences (e.g. a s Naturally occurring human sFLT-1 protein may include any suitable variants of sFLT-1, including, but not limited to functional fragments, sequences comprising insertions, deletions, substitutions, pseudofragments, pseudogenes, splice variants or artificially optimized sequences. In some cases, "sFLT-1 protein" may be at least about 90%, 91%, 92%, 93%, 94%, 95%, 96%, 97%, 98%, 99%, 99.9%, 99.99% or 100% homologous to the naturally occurring human sFLT-1 protein sequence. In some cases, "sFLT-1 protein" may be at most about 90%, 91%, 92%, 93%, 94%, 95%, 96%, 97%, 98%, 99%, 99.9%, 99.99% or 100% homologous to the naturally occurring human sFLT-1 protein sequence. In some cases, "sFLT-1 protein" may be at least about 90%>, 91%, 92%, 93%, 94%, 95%, 96%, 97%, 98%, 99%, 99.9%, 99.99% or 100% spatially homologous to the naturally occurring human sFLT-1 protein conformation. In some cases, "sFLT-1 protein" may be at most about 90%, 91%, 92%, 93%, 94%, 95%, 96%, 97%, 98%, 99%, 99.9%, 99.99% or 100% spatially homologous to the naturally occurring human sFLT-1 protein conformation.

Further, the soluble truncated form of the VEGF receptor FLT-1, sFLT-1, is the only known endogenous specific inhibitor of VEGF. In nature, it is generated by alternative mRNA splicing and lacks the membrane-proximal immunoglobulin-like domain, the transmembrane spanning region and the intracellular tyrosine-kinase domain. Structurally, FLT-1 and sFLT-1 protein may both comprise multiple functional domains. In some variants, FLT and sFLT proteins commonly share 6 interlinked domain; 3 domains involved in dimerization of the protein and 3 domains involved in the binding of a ligand, such as VEGF.

sFLT-1 is the only known endogenous specific inhibitor of VEGF. This interaction is specific and can be competed away with 100-fold excess unlabeled VEGF. In some cases, the angiostatic activity of sFLT-1 may result from inhibition of VEGF by two mechanisms: i) sequestration of VEGF, to which it binds with high affinity, and ii) formation of inactive heterodimers with membrane-spanning isoforms of the VEGF receptors FLTt-1 and FLK-l/KDR. As known in the art, in vitro binding assays have indicate that sFLT-1 binds VEGF with high affinity and may also inhibit VEGF driven proliferation of human umbilical vein endothelial cells. In animal models for cancer, sFLT-1 inhibits tumor growth. In some cases, sFLT-1 may function in a substoichiometric or dominant negative manner, as excess VEGF in the extracellular space may be prevented from binding and subsequently activating the VEGF receptor. These properties of sFLT-1 have been described in Kendall and Thomas, 1993; Proc Natl Acad Sci. 90: 10705-10709, which is incorporated herein by reference in its entirety. As is known in the art, functional fragments of sFLT-1 can be used in place of the full-length protein. More specifically, the VEGF binding domain (domain 2), or alternatively domain 2 of sFLT-1 plus domain 3 from sFLTl, KDR, or another family member, can be used to bind and inactivate VEGF. Such functional fragments are described in Wiesmann et al., 1997; Cell, 91 : 695-704, which is incorporated herein by reference in its entirety. The terms "sFLT-1" and "a functional fragment of sFLT-1" are equivalent and used here interchangeably.

Suitable gene product polypeptides that may be delivered according to the methods disclosed herein also include, e.g., retinoschisin, retinitis pigmentosa GTPase regulator (RGPR)-interacting protein-1 (see, e.g., GenBank Accession Nos. Q96KN7, Q9EPQ2, and Q9GLM3); peripherin-2 (Prph2) (see, e.g., GenBank Accession No. NP.sub.--000313; peripherin; a retinal pigment epithelium-specific protein (RPE65), (see, e.g., GenBank AAC39660; and Morimura et al. (1998) Proc. Natl. Acad. Sci. USA 95:3088); CHM (choroidermia (Rab escort protein 1)), a polypeptide that, when defective or missing, causes choroideremia (see, e.g., Donnelly et al. (1994) Hum. Mol. Genet. 3:1017; and van Bokhoven et al. (1994) Hum. Mol. Genet. 3:1041); and Crumbs homolog 1 (CRB1), a polypeptide that, when defective or missing, causes Leber congenital amaurosis and retinitis pigmentosa (see, e.g., den Hollander et al. (1999) Nat. Genet. 23:217; and GenBank Accession No. CAM23328).

Suitable polypeptides also include polypeptides that, when defective or missing, lead to achromotopsia, where such polypeptides include, e.g., cone photoreceptor cGMP-gated channel subunit alpha (CNGA3) (see, e.g., GenBank Accession No. NP.sub.--001289; and Booij et al. (2011) Ophthalmology 118:160-167); cone photoreceptor cGMP-gated cation channel beta-subunit (CNGB3) (see, e.g., Kohl et al. (2005) Eur J Hum Genet. 13(3):302); guanine nucleotide binding protein (G protein), alpha transducing activity polypeptide 2 (GNAT2) (ACHM4); and ACHM5; and polypeptides that, when defective or lacking, lead to various forms of color blindness (e.g., L-opsin, M-opsin, and S-opsin). See Mancuso et al. (2009) Nature 461(7265):784-787.

In some cases, a gene product of interest is a site-specific endonuclease that provide for site-specific knock-down of gene function, e.g., where the endonuclease knocks out an allele associated with a retinal disease. For example, where a dominant allele encodes a defective copy of a gene that, when wild-type, is a retinal structural protein and/or provides for normal retinal function, a site-specific endonuclease can be targeted to the defective allele and knock out the defective allele.

In addition to knocking out a defective allele, a site-specific nuclease can also be used to stimulate homologous recombination with a donor DNA that encodes a functional copy of the protein encoded by the defective allele. Thus, e.g., a subject rAAV virion can be used to deliver both a site-specific endonuclease that knocks out a defective allele, and can be used to deliver a functional copy of the defective allele, resulting in repair of the defective allele, thereby providing for production of a functional retinal protein (e.g., functional retinoschisin, functional RPE65, functional peripherin, etc.). See, e.g., Li et al. (2011) Nature 475:217. In some embodiments, a subject rAAV virion comprises a heterologous nucleotide sequence that encodes a site-specific endonuclease; and a heterologous nucleotide sequence that encodes a functional copy of a defective allele, where the functional copy encodes a functional retinal protein. Functional retinal proteins include, e.g., retinoschisin, RPE65, retinitis pigmentosa GTPase regulator (RGPR)-interacting protein-1, peripherin, peripherin-2, and the like.

Site-specific endonucleases that are suitable for use include, e.g., zinc finger nucleases (ZFNs); and transcription activator-like effector nucleases (TALENs), where such site-specific endonucleases are non-naturally occurring and are modified to target a specific gene. Such site-specific nucleases can be engineered to cut specific locations within a genome, and non-homologous end joining can then repair the break while inserting or deleting several nucleotides. Such site-specific endonucleases (also referred to as "INDELs") then throw the protein out of frame and effectively knock out the gene. See, e.g., U.S. Patent Publication No. 2011/0301073.

In some embodiments, a nucleotide sequence encoding a gene product is operably linked to a constitutive promoter. In other embodiments, a nucleotide sequence encoding a gene product of interest is operably linked to an inducible promoter. In some instances, a nucleotide sequence encoding a gene product of interest is operably linked to a tissue-specific or cell type-specific regulatory element. In certain embodiments, the promoter selected from cytomegalovirus (CMV) promoter, Rous sarcoma virus (RSV) promoter, MMT promoter, EF-1 alpha promoter, UB6 promoter, chicken beta-actin promoter, CAG promoter, RPE65 promoter and opsin promoter.

For example, in some instances, a nucleotide sequence encoding a gene product of interest is operably linked to a photoreceptor-specific regulatory element (e.g., a photoreceptor-specific promoter), e.g., a regulatory element that confers selective expression of the operably linked gene in a photoreceptor cell. Suitable photoreceptor-specific regulatory elements include, e.g., a rhodopsin promoter; a rhodopsin kinase promoter (Young et al. (2003) Ophthalmol. Vis. Sci. 44:4076); a beta phosphodiesterase gene promoter (Nicoud et al. (2007) J. Gene Med. 9:1015); a retinitis pigmentosa gene promoter (Nicoud et al. (2007) supra); an interphotoreceptor retinoid-binding protein (IRBP) gene enhancer (Nicoud et al. (2007) supra); an IRBP gene promoter (Yokoyama et al. (1992) Exp Eye Res. 55:225).

Recombinant viral vectors (e.g., rAAV virions) described herein, and optionally encapsulating polynucleotide cassettes of the present disclosure, may be produced using standard methodology. For example, in the case of rAAV virions, an AAV expression vector comprising a polynucleotide cassette may be introduced into a producer cell, followed by introduction of an AAV helper construct comprising comprising a polynucleotide sequence encoding a variant capsid protein disclosed herein, and where the helper construct includes AAV coding regions capable of being expressed in the producer cell and which complement AAV helper functions absent in the AAV vector. This is followed by introduction of helper virus and/or additional vectors into the producer cell, wherein the helper virus and/or additional vectors provide accessory functions capable of supporting efficient rAAV virus production. The producer cells are then cultured to produce rAAV. These steps are carried out using standard methodology. Replication-defective AAV virions comprising variant capsid proteins described herein are made by standard techniques known in the art using AAV packaging cells and packaging technology. Examples of these methods may be found, for example, in U.S. Pat. Nos. 5,436,146; 5,753,500, 6,040,183, 6,093,570 and 6,548,286, expressly incorporated by reference herein in their entirety. Further compositions and methods for packaging are described in Wang et al. (US 2002/0168342), also incorporated by reference herein in its entirety.

### METHODS

The present invention also provides methods of delivering a gene product to a cell or tissue, comprising administering a virus or viral vector described herein to the cell or tissue. Certain embodiments of the present invention relate to methods of transducing AAV vectors followed by expression of a transgene in the presence of neutralizing antibodies (nAbs) in a subject comprising administering to the subject a recombinant adeno -associated virus (rAAV) virion at an amount capable of at least partially evading nAbs in the subject.

In particular embodiments, the rAAV may be any of those described herein. In some instances, the rAAV can be a native AAV of serotype 1, 2, 3, 4, 5, 6, 7, 8, or 9. In some instances, the rAAV can be a chimeric AAV comprising proteins from at least two serotypes. In some cases, the rAAV can comprise a variant capsid protein. In some instances, the rAAV can comprise amino acid insertions, deletions, or substitutions relative to the sequence of a parent natural AAV. In some embodiments, the rAAV can comprise an amino acid insertion in a GH loop of a capsid protein. Examples of such insertions are described in US 9,193,956 and US 8,663,624, the disclosure of each are incorporated by reference herein in their entirety.

Described herein is development of novel AAV vectors for clinical indications with IVT delivery that can overcome current limitations. AAV2.7m8 (described in US 9,193,956, the disclosure of which is incorporated by reference herein in its entirety) is a novel variant of AAV2 that can effectively transduce multiple layers of the non-human primate (NHP) retina following IVT injection. However, pre-existing nAbs could limit the effectiveness of AAV2.7m8 following IVT administration.

The present invention provides new methods of administration of viral vectors, e.g., AAV vectors, that are demonstrated herein to successfully transduce ocular cells in the presence of neutralizing antibodies that bind the AAV vector. In particular embodiments of any of the methods described herein, the administration is performed by ocular administration, retinal administration, subretinal administration and/or intravitreal administration. In particular embodiments, the administration is performed by ocular injection, retinal injection, subretinal injection and/or intravitreal injection. In certain embodiments, the subject being administered the viral vector has nAbs, e.g., nAbs that bind and neutralize AAV vectors, due to previous exposure to AAV. In certain embodiments, the subject being administered the viral vector has nAbs to the viral vector due to a previous administration of the viral vector to the subject. For example, a subject may have been previously administered an rAAV by IVT, which provoked an immune response and the production of nAbs that bind and neutralize the rAAV, thus potentially limiting the ability to successfully administer additional rAAV to further treat an ocular disease or disorder.

In one embodiment, the disclosure includes a method of transducing AAV for expression of a transgene in the presence of neutralizing antibodies (nAbs) in a subject comprising administering to the subject a recombinant adeno-associated virus (rAAV) virion at an amount capable of at least partially evading nAbs in the subject, wherein the rAAV can overcome nAbs and transduce a retinal cell in the presence of an amount of nAbs. In particular embodiments, the rAAV virion is AAV2.7m8. In particular embodiments, the subject is a mammalian subject. In various embodiments, the rAAV virion administered is at least about 1× 10¹¹vg, at least about 5×10¹¹ vg, at least about 1×10¹³ vg, at least about 5×10¹³ vg, at least about 1×10¹⁴ vg, at least about 5×10¹⁴ vg, at least about 1×10¹⁵ vg, at least about 5×10¹⁵ vg, or at least about 1×10¹⁶ vg. In certain embodiments, the amount of neutralizing antibodies is between about 0.46 mg/mL to about 1.85 mg/mL. In particular embodiments, the administration is performed by ocular administration, retinal administration, subretinal administration and/or intravitreal administration. In particular embodiments, the administration is performed by ocular injection, retinal injection, subretinal injection and/or intravitreal injection. In particular embodiments, the administration is an intravitreal injection. In certain embodiments, the subject is administered a first dose and a second dose of the rAAV virion, each administered dose comprising an amount of rAAV virion capable of atleast partially evading nAbs in the subject and transducing a retinal cell in the present of an amount of the nAbs.

In one embodiment, the disclosure includes a method of delivering a transgene encoding a gene product to an ocular cell in a subject, comprising administering to one or more sites within the eye of the subject an effective amount of a rAAV comprising the transgene. In certain embodiments, the transgene is expressed in the presence of nAbs in the subject that specifically bind the rAAV, and the effective amount of the rAAV administered is sufficient to at least partially evade the nAbs in the subject and transduce the ocular cell, wherein the rAAV-transduced ocular cell expresses the gene product. In particular embodiments, the rAAV virion is AAV2.7m8. In particular embodiments, the subject is a mammalian subject. In particular embodiments, the administration is performed by ocular administration, retinal administration, subretinal administration and/or intravitreal administration. In particular embodiments, the administration is performed by ocular injection, retinal injection, subretinal injection and/or intravitreal injection. In particular embodiments, the administration is an intravitreal injection.

In various embodiments of the methods described herein, the effective amount of the rAAV administered is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, at least about 1×10¹⁴ vg, at least about 5×10¹⁴ vg, at least about 1×10¹⁵ vg, at least about 5×10¹⁵ vg, or at least about 1×10¹⁶ vg. In some embodiments, the effective amount of the rAAV administered is about 1×10¹¹ vg to about 5×10¹² vg, or about 1×10¹¹ vg to about 1×10¹⁴ vg.

In particular embodiments, an effective amount of rAAV administered to a subject that does not comprise nAbs, or a first effective amount, is at least about 1×10¹⁰ vg, at least about 1×10¹¹ vg, at least about 5×10¹¹ vg, at least about 1×10¹² vg, at least about 5×10¹² vg, or at least about 1 × 10¹³ vg. In some embodiments, the effective amount of the rAAV administered is about 1×10¹¹ vg to about 5×10¹² vg, or about 1×10¹¹ vg to about 1×10¹³ vg. In some embodiments, the effective amount of the rAAV administered is at least about 1 × 10¹³ vg, at least about 5×10¹³ vg, at least about 1×10¹⁴ vg, at least about 5×10¹⁴ vg, at least about 1×10¹⁵ vg, at least about 5×10¹⁵ vg, or at least about 1×10¹⁶ vg.

In particular embodiments, an effective amount of rAAV administered to a subject comprising nAbs, or a second effective amount, is at least about 5×10¹² vg, at least about 1 × 10¹³ vg, at least about 5×10¹³ vg, or at least about 1×10¹⁴ vg, at least about 5×10¹⁴ vg, or at least about 1×10¹⁵ vg. In some embodiments, the effective amount of the rAAV administered is about 1×10¹² vg to about 5×10¹⁴ vg, or about 5×10¹² vg to about 1×10¹⁴ vg.

In certain embodiments, the concentration of nAbs in the subject is about 0.46 mg/mL up to about 0.92 mg/mL, or about 0.46 mg/mL up to about 1.38 mg/mL, or about 0.46 mg/mL up to about 1.85 mg/mL, or about 0.46 mg/mL up to about 2.31 mg/mL, or about .1 mg/mL to about 5 mg/mL, or about .2 mg/mL to about 4 mg/mL, or about .5 mg/mL to about 3 mg.mL at one or more of the one or more sites of administration.

In certain embodiments, the rAAV is administered retinally, subretinally, and/or intravitreally.

In one embodiment, the disclosure includes a method of delivering a transgene encoding a gene product to an ocular cell in a mammal, e.g., a human, comprising administering to the mammal an effective amount of a rAAV comprising the transgene by IVT, where the subject comprises nAbs that specifically bind the rAAV, and the effective amount of the rAAV administered is sufficient to at least partially evade the nAbs in the subject and transduce the ocular cell. In particular embodiments, the rAAV virion is AAV2.7m8. In particular embodiments, the effective amount of rAAV is at least at least about 5×10¹² vg, at least about 1×10¹³ vg, at least about 5×10¹³ vg, or at least about 1×10¹⁴ vg, at least about 5×10¹⁴ vg, or at least about 1×10¹⁵ vg. In some embodiments, the effective amount of the rAAV administered is about 1×10¹² vg to about 5×10¹⁴ vg, or about 5×10¹² vg to about 1×10¹⁴ vg. In some embodiments, the effective amount of the rAAV administered is at least about 1 × 10¹³ vg, at least about 5×10¹³ vg, at least about 1×10¹⁴ vg, at least about 5×10¹⁴ vg, at least about 1×10¹⁵ vg, at least about 5×10¹⁵ vg, or at least about 1×10¹⁶ vg. In particular embodiments, the administration is performed by ocular administration, retinal administration, subretinal administration and/or intravitreal administration. In particular embodiments, the administration is performed by ocular injection, retinal injection, subretinal injection and/or intravitreal injection. In particular embodiments, the administration is an intravitreal injection.

In another embodiment, the disclosure includes a method for treating an ocular disease or disorder in a subject, comprising administering to one or more site within the eye of the subject an effective amount of a rAAV comprising a transgene encoding a therapeutic gene product. In certain embodiments, the transgene is expressed in the presence of nAbs that specifically bind the rAAV, and the effective amount of the rAAV administered is an amount sufficient to at least partially evade the nAbs in the subject and transduce ocular cells within the subject, and wherein the transduced ocular cells express the therapeutic gene product. In particular embodiments, the rAAV virion is AAV2.7m8. In particular embodiments, the subject is a mammalian subject. In various embodiments, the effective amount of the rAAV administered is at least about 1×10¹⁰ vg, at least about 1×10¹¹ vg, at least about 5×10¹¹ vg, at least about 1×10¹² vg, at least about 5×10¹² vg, at least about 1 × 10¹³ vg, at least about 5×10¹³ vg, or at least about 1×10¹⁴ vg. In some embodiments, the effective amount of the rAAV administered is about 1×10¹¹ vg up to about 5×10¹² vg, or about 1×10¹¹ vg up to about 1×10¹⁴ vg. In particular embodiments, an effective amount of rAAV administered to a subject that does not comprise nAbs, or a first effective amount, is at least about 1×10¹⁰ vg, at least about 1×10¹¹ vg, at least about 5×10¹¹ vg, at least about 1×10¹² vg, at least about 5×10¹² vg, or at least about 1×10¹³ vg. In some embodiments, the effective amount of the rAAV administered is about 1×10¹¹ vg to about 5×10¹² vg, or about 1×10¹¹ vg to about 1×10¹³ vg. In some embodiments, the effective amount of the rAAV administered is at least about 1 × 10¹³ vg, at least about 5×10¹³ vg, at least about 1×10¹⁴ vg, at least about 5×10¹⁴ vg, at least about 1×10¹⁵ vg, at least about 5×10¹⁵ vg, or at least about 1×10¹⁶ vg.

In particular embodiments, an effective amount of rAAV administered to a subject comprising nAbs, or a second effective amount, is at least about 5×10¹² vg, at least about 1 × 10¹³ vg, at least about 5×10¹³ vg, or at least about 1×10¹⁴ vg, at least about 5×10¹⁴ vg, at least about 1×10¹⁵ vg at least about 5×10¹⁵ vg, or at least about 1×10¹⁶ vg. In some embodiments, the effective amount of the rAAV administered is about 1×10¹² vg to about 5×10¹⁴ vg, or about 5×10¹² vg to about 1×10¹⁴ vg. In certain embodiments, the concentration of nAbs in the subject is about 0.46 mg/mL up to about 0.92 mg/mL, or about 0.46 mg/mL up to about 1.38 mg/mL, or about 0.46 mg/mL up to about 1.85 mg/mL, or about 0.46 mg/mL up to about 2.31 mg/mL, or about .1 mg/mL to about 5 mg/mL, or about .2 mg/mL to about 4 mg/mL, or about .5 mg/mL to about 3 mg.mL at one or more of the one or more sites of administration. In certain embodiments, the rAAV is administered ocularly, retinally, subretinally, and/or intravitreally.

In certain embodiments of any of the methods described here, the ocular disease or disorder is glaucoma, retinitis pigmentosa, macular degeneration, retinoschisis, Leber's Congenital Amaurosis, diabetic retinopathy, achromotopsia, or color blindness. In some embodiments, the ocular disease is macular degeneration. In specific embodiments, the macular degeneration is wet macular degeneration. In other embodiments, the macular degeneration is dry macular degeneration.

In certain embodiments, the gene product delivered by methods of the present invention may be any therapeutic gene product, including but not limited to any gene products disclosed herein. In some embodiments of any of the methods described here, the gene product is an anti-angiogenic polypeptide. In other embodiments, the gene product is a vascular endothelial growth factor (VEGF)-binding protein. In certain embodiments, the gene product is an anti-VEGF agent or anti-VEGF protein. In certain embodiments, the gene product is an opsin protein.

In particular embodiments, the methods are used to deliver a gene product that is an anti-VEGF agent for the treatment of an angiogenic or neovascular disorder, including those associated with cancer, ischemia, and inflammation. In particular embodiments, a subject is administered a virus encoding an anti-VEGF agent to treat an eye disease or disorder, including but not limited to: ischemic retinopathy, intraocular neovascularization, age-related macular degeneration (AMD), wet-AMD, dry-AMD, retinal neovascularization, diabetic macular edema, diabetic retina ischemia, diabetic retinal edema, proliferative diabetic retinopathy, retinal vein occlusion, central retinal vein occlusion, branched retinal vein occlusion.

Suitable gene product polypeptides that may be delivered according to the methods disclosed herein and related diseases or disorders that they may be used to treat also include, e.g., retinoschisin, retinitis pigmentosa GTPase regulator (RGPR)-interacting protein-1 (see, e.g., GenBank Accession Nos. Q96KN7, Q9EPQ2, and Q9GLM3); peripherin-2 (Prph2) (see, e.g., GenBank Accession No. NP.sub.--000313; peripherin; a retinal pigment epithelium-specific protein (RPE65), (see, e.g., GenBank AAC39660; and Morimura et al. (1998) Proc. Natl. Acad. Sci. USA 95:3088); CHM (choroidermia (Rab escort protein 1)), a polypeptide that, when defective or missing, causes choroideremia (see, e.g., Donnelly et al. (1994) Hum. Mol. Genet. 3:1017; and van Bokhoven et al. (1994) Hum. Mol. Genet. 3:1041); and Crumbs homolog 1 (CRB1), a polypeptide that, when defective or missing, causes Leber congenital amaurosis and retinitis pigmentosa (see, e.g., den Hollander et al. (1999) Nat. Genet. 23:217; and GenBank Accession No. CAM23328).

Suitable polypeptides also include polypeptides that, when defective or missing, lead to achromotopsia, where such polypeptides include, e.g., cone photoreceptor cGMP-gated channel subunit alpha (CNGA3) (see, e.g., GenBank Accession No. NP.sub.--001289; and Booij et al. (2011) Ophthalmology 118:160-167); cone photoreceptor cGMP-gated cation channel beta-subunit (CNGB3) (see, e.g., Kohl et al. (2005) Eur J Hum Genet. 13(3):302); guanine nucleotide binding protein (G protein), alpha transducing activity polypeptide 2 (GNAT2) (ACHM4); and ACHM5; and polypeptides that, when defective or lacking, lead to various forms of color blindness (e.g., L-opsin, M-opsin, and S-opsin). See Mancuso et al. (2009) Nature 461(7265):784-787.

In some cases, a gene product of interest is a site-specific endonuclease that provide for site-specific knock-down of gene function, e.g., where the endonuclease knocks out an allele associated with a retinal disease. For example, where a dominant allele encodes a defective copy of a gene that, when wild-type, is a retinal structural protein and/or provides for normal retinal function, a site-specific endonuclease can be targeted to the defective allele and knock out the defective allele.

In addition to knocking out a defective allele, a site-specific nuclease can also be used to stimulate homologous recombination with a donor DNA that encodes a functional copy of the protein encoded by the defective allele. Thus, e.g., a subject rAAV virion can be used to deliver both a site-specific endonuclease that knocks out a defective allele, and can be used to deliver a functional copy of the defective allele, resulting in repair of the defective allele, thereby providing for production of a functional retinal protein (e.g., functional retinoschisin, functional RPE65, functional peripherin, etc.). See, e.g., Li et al. (2011) Nature 475:217. In some embodiments, a subject rAAV virion comprises a heterologous nucleotide sequence that encodes a site-specific endonuclease; and a heterologous nucleotide sequence that encodes a functional copy of a defective allele, where the functional copy encodes a functional retinal protein. Functional retinal proteins include, e.g., retinoschisin, RPE65, retinitis pigmentosa GTPase regulator (RGPR)-interacting protein-1, peripherin, peripherin-2, and the like.

Site-specific endonucleases that are suitable for use include, e.g., zinc finger nucleases (ZFNs); and transcription activator-like effector nucleases (TALENs), where such site-specific endonucleases are non-naturally occurring and are modified to target a specific gene. Such site-specific nucleases can be engineered to cut specific locations within a genome, and non-homologous end joining can then repair the break while inserting or deleting several nucleotides. Such site-specific endonucleases (also referred to as "INDELs") then throw the protein out of frame and effectively knock out the gene. See, e.g., U.S. Patent Publication No. 2011/0301073.

In certain embodiments, methods disclosed herein are used to provide multiple administrations of a viral vector, e.g., rAAV, to a subject, e.g., by IVT. As demonstrated herein, the present methods are able to successfully transduce ocular cells by IVT in the presence of nAbs. Accordingly, the methods may be employed to successfully deliver a gene product by IVT to ocular cells within a subject who has such nAbs.

In one embodiment, the disclosure includes a method for treating an ocular disease or disorder in a subject comprising: first, administering to a first eye of the subject a first effective amount of a first viral vector (e.g., rAAV) comprising a first transgene encoding a first gene product (i.e., a first dose), next, waiting for a period of time, and then, administering to a second eye of the subject a second effective amount of a second viral vector (e.g., rAAV) comprising a second transgene encoding a second gene product (i.e., a second dose), wherein the first and second effective amounts of viral vector (e.g., rAAV) are amounts sufficient to transduce cells of the eye, wherein the transduced cells express the first and second gene products. In certain embodiments, the method may further comprise subsequent administrations of a viral vector following additional periods of time. In particular embodiments, the methods may comprise two or more, three or more, four or more, or five or more administrations of a viral vector with periods of time between each administration. In particular embodiments, the administration is performed by ocular administration, retinal administration, subretinal administration and/or intravitreal administration. In particular embodiments, each administration is independently performed by ocular injection, retinal injection, subretinal injection and/or intravitreal injection. In particular embodiments, the administration is an intravitreal injection.

In specific embodiments, the first viral vector (e.g., rAAV) and the second viral vector (e.g., rAAV) are of the same serotype. In other embodiments, the first and second viral vectors (e.g., rAAVs) comprise the same capsid proteins. In certain embodiments, the first rAAV and the second rAAV are both AAV, e.g., AAV2.7m8. In some embodiments, the first and second viral vectors (e.g., rAAVs) are different serotypes.

In certain embodiments, the period of time between administering the first viral vector (e.g., rAAV) and administering the second viral vector (e.g., rAAV) is at least about one week, at least about one month, at least about three months, at least about six months, at least about one year, at least about 18 months, at least about three years, or longer than about three years. In some embodiments, the subject is not administered a rAAV during the period of time between administering the first rAAV and administering the second rAAV. In certain embodiments, the subject may receive one, two, three, four, or more effective amounts of rAAV in either one or both eyes, following the second administration of rAAV. In other embodiments, the subject does not receive any additional administrations of rAAV after the second administration of rAAV.

In certain embodiments of any of the methods described herein that comprise administering a first dose (i.e., a first effective amount) and a second dose (i.e., a second effective amount) of the rAAV, the first dose and second dose are administered to the same eye. In certain embodiments, the first dose and second dose are administered to different eyes. In particular embodiments, the first and second dose are administered for treating the same ocular disease. In certain embodiments, the first and second dose are administered for treating different ocular diseases. In certain embodiments, the first dose and second dose comprise rAAV comprising the same heterologous polynucleotide encoding a gene product. In particular embodiments, the first dose and second dose comprise rAAV comprising different heterologous polynucleotides, e.g., encoding different gene products.

In some embodiments of any of the methods described here that comprise administering a first dose and a second dose of the rAAV, the effective amount of the rAAV administered in the first effective dose is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, at least about 1×10¹⁴ vg, at least about 5×10¹⁵ vg, at least about 1×10¹⁵ vg, at least about 5×10¹⁵ vg, or at least about 1×10¹⁶ vg. In some embodiments, the effective amount of the rAAV administered in the first effective dose is about 1×10¹¹ vg up to about 5×10¹² vg, or about 1×10¹¹ vg up to about 1×10¹⁴ vg. In certain embodiments, the effective amount of the rAAV administered in the second effective dose is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, or at least about 1×10¹⁴ vg. In some embodiments, the effective amount of the rAAV administered in the second effective dose is about 1×10¹¹ vg up to about 5×10¹² vg, or about 1×10¹¹ vg up to about 1×10¹⁴ vg. In particular embodiments, an effective amount of rAAV administered to a subject that does not comprise nAbs, or a first effective amount, is at least about 1×10¹⁰ vg, at least about 1×10¹¹ vg, at least about 5×10¹¹ vg, at least about 1×10¹² vg, at least about 5×10¹² vg, or at least about 1 × 10¹³ vg. In some embodiments, the effective amount of the rAAV administered is about 1×10¹¹ vg to about 5×10¹² vg, or about 1×10¹¹ vg to about 1×10¹³ vg. In particular embodiments, an effective amount of rAAV administered to a subject comprising nAbs, or a second effective amount, is at least about 5×10¹² vg, at least about 1 × 10¹³ vg, at least about 5×10¹³ vg, or at least about 1×10¹⁴ vg, at least about 5×10¹⁴ vg, or at least about 1×10¹⁵ vg. In some embodiments, the effective amount of the rAAV administered is about 1×10¹² vg to about 5×10¹⁴ vg, or about 5×10¹² vg to about 1×10¹⁴ vg. In certain embodiments, the concentration of nAbs in the subject is about 0.46 mg/mL up to about 0.92 mg/mL, or about 0.46 mg/mL up to about 1.38 mg/mL, or about 0.46 mg/mL up to about 1.85 mg/mL, or about 0.46 mg/mL up to about 2.31 mg/mL, or about .1 mg/mL to about 5 mg/mL, or about .2 mg/mL to about 4 mg/mL, or about .5 mg/mL to about 3 mg.mL at one or more of the one or more sites of administration. In certain embodiments, the rAAV is administered retinally, subretinally, and/or intravitreally. In particular embodiments, it is delivered by IVT.

In specific embodiments, the first effective amount of the rAAV administered is the same as the second effective amount of the rAAV administered. In other embodiments, the first effective amount of the rAAV administered is higher than the second effective amount of the rAAV administered. In some embodiments, the first effective amount of the rAAV administered is lower than the second effective amount of the rAAV administered. In certain embodiments, it is contemplated that lower dose is used for the first administration, particularly if the subject being treated does not comprise nAbs, whereas a higher dose is used for the second or any subsequent administration, particularly if the subject has developed nAbs following administration of the first dose.

In one embodiment, the present disclose includes a method of treating an ocular disease or disorder, e.g., a disease associated with aberrant angiogenesis, in a subject in need thereof, comprising the following:
(1) administering to one or both eyes of the subject by ocular injection (e.g., retinal, subretinal and/or intravitreal injection) an effective amount of a rAAV comprising a transgene encoding a therapeutic gene product, wherein at least a plurality of ocular cells (e.g., retinal cells) of the one or both eyes are transduced and express the therapeutic gene product;
(2) waiting for a period of time; and
(3) administering to one or both eyes of the subject by ocular injection (e.g., retinal, subretinal and/or intravitreal injection) an effective amount of a rAAV comprising a transgene encoding a therapeutic gene product, wherein at least a plurality of ocular cells (e.g., retinal cells) of the one or both eyes are transduced and express the therapeutic gene product. In certain embodiments, step (1) and/or step (3) are performed by IVT.

In certain embodiments, the therapeutic gene product administered at step (1) and/or step (3) is an anti-VEGF agent or protein, e.g., sFlt or aflibercept. In certain embodiments, the therapeutic gene product administered at step (1) is the same as the therapeutic gene product administered at step (3). In certain embodiments, the disease or disorder being treated is associated with aberrant angiogenesis in the eye. In particular embodiments, the disease or disorder is macular degeneration, e.g., age-related macular degeneration (AMD). In particular embodiments, it is wet macular degeneration or dry macular degeneration.

In other embodiments, the methods are practiced to deliver any of the gene products disclosed herein.

In particular embodiments, the rAAV administered in step (1) and/or step (3) is rAA V2.7m8.

In certain embodiments, the time period of step (b) is at least one week, at least one month, at least three months, at least six months, at least one year, at least 18 months, at least two years, at least three years, or longer than three years.

In particular embodiments, the effective amount administered in step (1) is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, or at least about 1×10¹⁴ vg. at least about 5×10¹⁵ vg, or at least about 1×10¹⁶ vg. In some embodiments, the effective amount of the rAAV administered in the first effective dose is about 1×10¹¹ vg up to about 5×10¹² vg, or about 1×10¹¹ vg up to about 1×10¹⁴ vg.

In particular embodiments, the effective amount administered in step (3) is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, at least about 1×10¹⁴ vg, at least about 5×10¹⁵ vg, at least about 1×10¹⁵ vg, at least about 5×10¹⁵ vg, or at least about 1×10¹⁶ vg. In certain embodiments, the effective amount of the rAAV administered in the second effective dose is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, or at least about 1×10¹⁴ vg. In some embodiments, the effective amount of the rAAV administered in the second effective dose is about 1×10¹¹ vg up to about 5×10¹² vg, or about 1×10¹¹ vg up to about 1×10¹⁴ vg.

In particular embodiments, an effective amount of rAAV administered to a subject that does not comprise nAbs, or a first effective amount, is at least about 1×10¹⁰ vg, at least about 1×10¹¹ vg, at least about 5×10¹¹ vg, at least about 1×10¹² vg, at least about 5×10¹² vg, or at least about 1 × 10¹³ vg. In some embodiments, the effective amount of the rAAV administered is about 1×10¹¹ vg to about 5×10¹² vg, or about 1×10¹¹ vg to about 1×10¹³ vg. In particular embodiments, an effective amount of rAAV administered to a subject comprising nAbs, or a second effective amount, is at least about 5×10¹² vg, at least about 1 × 10¹³ vg, at least about 5×10¹³ vg, or at least about 1×10¹⁴ vg, at least about 5×10¹⁴ vg, or at least about 1×10¹⁵ vg. In some embodiments, the effective amount of the rAAV administered is about 1×10¹² vg to about 5×10¹⁴ vg, or about 5×10¹² vg to about 1×10¹⁴ vg.

In certain embodiments, the subject comprises nAbs that bind and are capable of neutralizing the rAAV before step (a) and/or before step (c). In particular embodiments, the concentration of nAbs in the subject is about 0.46 mg/mL up to about 0.92 mg/mL, or about 0.46 mg/mL up to about 1.38 mg/mL, or about 0.46 mg/mL up to about 1.85 mg/mL, or about 0.46 mg/mL up to about 2.31 mg/mL, or about .1 mg/mL to about 5 mg/mL, or about .2 mg/mL to about 4 mg/mL, or about .5 mg/mL to about 3 mg/mL at one or more of the one or more sites of administration.

In some embodiments of any of the methods described here that comprise administering a first dose and a second dose of the rAAV, the effective amount of the rAAV administered in the first effective dose is about 1×10¹¹ vg up to about 5×10¹² vg, or about 1×10¹¹ vg up to about 1×10¹⁴ vg. In certain embodiments, the effective amount of the rAAV administered in the second effective dose is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, or at least about 1×10¹⁴ vg. In some embodiments, the effective amount of the rAAV administered in the second effective dose is about 1×10¹¹ vg up to about 5×10¹² vg, or about 1×10¹¹ vg up to about 1×10¹⁴ vg. In particular embodiments, an effective amount of rAAV administered to a subject that does not comprise nAbs, or a first effective amount, is at least about 1×10¹⁰ vg, at least about 1×10¹¹ vg, at least about 5×10¹¹ vg, at least about 1×10¹² vg, at least about 5×10¹² vg, or at least about 1 × 10¹³ vg. In some embodiments, the effective amount of the rAAV administered is about 1×10¹¹ vg to about 5×10¹² vg, or about 1×10¹¹ vg to about 1×10¹³ vg. In particular embodiments, an effective amount of rAAV administered to a subject comprising nAbs, or a second effective amount, is at least about 5×10¹² vg, at least about 1 × 10¹³ vg, at least about 5×10¹³ vg, or at least about 1×10¹⁴ vg, at least about 5×10¹⁴ vg, or at least about 1×10¹⁵ vg. In some embodiments, the effective amount of the rAAV administered is about 1×10¹² vg to about 5×10¹⁴ vg, or about 5×10¹² vg to about 1×10¹⁴ vg. In certain embodiments, the concentration of nAbs in the subject is about 0.46 mg/mL up to about 0.92 mg/mL, or about 0.46 mg/mL up to about 1.38 mg/mL, or about 0.46 mg/mL up to about 1.85 mg/mL, or about 0.46 mg/mL up to about 2.31 mg/mL, or about .1 mg/mL to about 5 mg/mL, or about .2 mg/mL to about 4 mg/mL, or about .5 mg/mL to about 3 mg.mL at one or more of the one or more sites of administration.

In certain embodiments of any of the methods described herein, the therapeutic gene product is an anti-VEGF agent or protein.

In certain embodiments of any of the methods disclosed herein, the disease or disorder being treated is associated with aberrant angiogenesis in the eye. In particular embodiments, the disease or disorder is macular degeneration, e.g., age-related macular degeneration (AMD). In particular embodiments, it is wet macular degeneration or dry macular degeneration.

In certain embodiments of any of the methods described here that comprise administering a first dose and a second dose of the rAAV, the first effective amount and the second effective amount of rAAV administered is an amount sufficient to at least partially evade nAbs in the subject and transduce the ocular cell, and the transduced ocular cell expresses the gene product.

In some embodiments of any of the methods described herein, the subject is not administered an immunosuppressant prior to, concurrent with, or following the administration of the first rAAV. In other embodiments, the subject is administered an immunosuppressant prior to, concurrent with, or following administration of the first rAAV. In specific embodiments, the subject is not administered an immunosuppressant after the administration of the first rAAV. In certain embodiments, an immunosuppressant is administered to the subject after the administration of the first rAAV and before or concurrent with the administration of the second rAAV. In some embodiments, and immunosuppressant is administered is administered to the subject after the administration of the second rAAV.

In some embodiments of any of the methods described here that comprise administering a first dose and a second dose of the rAAV, the administering of the first effective amount and the administering of the second effective amount is by intraocular injection or intravitreal injection. In other embodiments, the administration of the second effective amount of the second rAAV provides a therapeutic effect in the eye of the subject. In certain embodiments, the administration of the first effective amount and the second effective amount are to the same eye of the subject. In other embodiments, the administration of the first effective amount and the second affective amount are to different eyes of the subject. In specific embodiments, the first effective amount and the second effective amount are treating the same ocular disease or disorder. In some embodiments, the first effective amount and the second effective amount are treating different ocular diseases or disorders. In certain embodiments, the first gene product and the second gene product are the same. In other embodiments, the first gene product and the second gene product are different.

In certain embodiments of any of the methods disclosed herein for treating an ocular disease or disorder, the first effective amount of viral vector and the second effective amount of viral vectors are amounts sufficient to transduce at least a plurality of ocular cells, resulting in the expression of a therapeutically effective amount of the gene product, i.e., an amount sufficient to treat the disease or disorder.

In certain embodiments of any of the methods described herein, the method further comprises determining whether the subject comprises nAbs that bind and are capable of neutralizing the viral vector to be administered to the subject before such administration. Methods of determining whether a subject comprises nAbs are routine and known in the art. Where it is determined that the subject comprises such nAbs, the subject may be administered a higher dose of the viral vector, e.g., at least 1×10¹², at least 5×10¹² , at least 1×10¹³, at least 5×10¹3 or at least 1×10¹⁴ viral genomes (vgs).

Any of the viruses or viral vectors described herein may present in a pharmaceutical composition and/or administered to a subject while in a pharmaceutical composition. A pharmaceutical composition is a formulation containing one or more active ingredients as well as one or more excipients, carriers, stabilizers or bulking agents, which is suitable for administration to a human patient to achieve a desired diagnostic result or therapeutic or prophylactic effect. For storage stability and convenience of handling, a pharmaceutical composition can be formulated as a lyophilized (i.e. freeze-dried) or vacuum dried powder which can be reconstituted with saline or water prior to administration to a patient

The vector or recombinant viruses (virions) can be incorporated into pharmaceutical compositions for administration to mammalian patients, particularly humans. The vector or virions can be formulated in nontoxic, inert, pharmaceutically acceptable aqueous carriers, preferably at a pH ranging from 3 to 8, more preferably ranging from 6 to 8. Such sterile compositions will comprise the vector or virion containing the nucleic acid encoding the therapeutic molecule dissolved in an aqueous buffer having an acceptable pH upon reconstitution.

In some aspects, the pharmaceutical composition provided herein comprise a therapeutically effective amount of a vector or virion in admixture with a pharmaceutically acceptable carrier and/or excipient, for example saline, phosphate buffered saline, phosphate and amino acids, polymers, polyols, sugar, buffers, preservatives and other proteins. Exemplary amino acids, polymers and sugars and the like are octylphenoxy polyethoxy ethanol compounds, polyethylene glycol monostearate compounds, polyoxyethylene sorbitan fatty acid esters, sucrose, fructose, dextrose, maltose, glucose, mannitol, dextran, sorbitol, inositol, galactitol, xylitol, lactose, trehalose, bovine or human serum albumin, citrate, acetate, Ringer's and Hank's solutions, cysteine, arginine, carnitine, alanine, glycine, lysine, valine, leucine, polyvinylpyrrolidone, polyethylene and glycol. Preferably, this formulation is stable for at least six months at 4° C.

In some aspects, the pharmaceutical composition provided herein comprises a buffer, such as phosphate buffered saline (PBS) or sodium phosphate/sodium sulfate, tris buffer, glycine buffer, sterile water and other buffers known to the ordinarily skilled artisan such as those described by Good et al. (1966) Biochemistry 5:467. The pH of the buffer in which the pharmaceutical composition comprising the anti-VEGF contained in the adenoviral vector delivery system, may be in the range of 6.5 to 7.75, 7 to 7.5, or 7.2 to 7.4. The pH of the formulation may range from about 3.0 to about 12.0. The pH of the immunogenic composition may be at least about 3, 4, 5, 6, 7, 8, 9, 10, 11 or 12 pH units. The pH of the immunogenic composition may be at most about 3, 4, 5, 6, 7, 8, 9, 10, 11 or 12 pH units.

The present invention further provides unit dosage forms of a viral vector or virus described herein. In particular embodiments, an amount the virus or viral vector may be measured as vector genomes. In some cases, the unit dose of the pharmaceutical composition of the disclosure is 1×10¹⁰to 3×10¹² vector genomes. In some cases, the unit dose of the pharmaceutical composition of the disclosure is 1×10⁹to 3×10¹³vector genomes. In some cases, the unit dose of the pharmaceutical composition of the disclosure is 1×10¹⁰to 1×1¹¹ vector genomes. In some cases, the unit dose of the pharmaceutical composition of the disclosure is 1×10⁸to 3 ×10¹⁴ vector genomes. In some cases, the unit dose of the pharmaceutical composition of the disclosure is at least about 1×10¹, 1×10², 1×10³ 1×10⁴, 1×10⁵, 1×10⁶, 1×10⁷ 1×10⁸_{,} 1×10⁹, 1×10¹⁰, 1×10¹¹, 1×10¹², 1×10¹³, 1×10¹⁴, 1×10¹⁵, 1×10¹⁶, 1×10¹⁷ and 1×10¹⁸vector genomes. In some cases, the unit dose of the pharmaceutical composition of the disclosure is 1×10⁸to 3×10¹⁴ vector genomes. In some cases, the unit dose of the pharmaceutical composition of the disclosure is at most about 1×10¹, 1×10% 1×10³ 1×10⁴, 1×10⁵, 1×10⁶, 1×10⁷, 1×10⁸, 1×10⁹, 1×10¹⁰, 1×10¹¹, 1×10¹³, 1×10¹³, 1×10¹⁴, 1×10¹⁵, 1×10¹⁶, 1×10¹⁷ and 1×10¹⁸vector genomes. In some embodiments the unit dose of the pharmaceutical composition comprises at least about 1×10¹⁰ vg, at least about 1×10¹¹ vg, at least about 5×10¹¹vg, at least about 1×10¹² vg, at least about 5×10¹² vg, at least about 1 × 10¹³ vg, at least about 5×10¹³ vg, or at least about 1×10¹⁴ vg. In some embodiments, the unit dose is about 1×10¹¹ vg up to about 5×10¹² vg, or about 1×10¹¹ vg up to about 1×10¹⁴ vg. In certain embodiments, the unit dose is at least about 1×10¹⁰ vg, at least about 1×10¹¹ vg, at least about 5×10¹¹ vg, at least about 1×10¹² vg, at least about 5×10¹² vg, at least about 1×10¹³ vg, at least about 5×10¹³ vg, or at least about 1×10¹⁴ vg. In some embodiments, the unit dose is about 1×10¹¹ vg up to about 5×10¹² vg, or about 1×10¹¹ vg up to about 1×10¹⁴ vg. In particular embodiments, the unit dose is at least about 1×10¹⁰ vg, at least about 1×10¹¹vg, at least about 5×10¹¹ vg, at least about 1×10¹² vg, at least about 5×10¹² vg, or at least about 1×10¹³ vg. In some embodiments, the unit dose is about 1×10¹¹ vg to about 5×10¹² vg, or about 1×10¹¹ vg to about 1×10¹³ vg. In particular embodiments, the unit dose is at least about 5×10¹² vg, at least about 1×10¹³ vg, at least about 5×10¹³ vg, or at least about 1×10¹⁴ vg, at least about 5×10¹⁴ vg, or at least about 1×10¹⁵ vg. In some embodiments, the unit dose is about 1×10¹² vg to about 5×10¹⁴ vg, or about 5×10¹² vg to about 1×10¹⁴ vg.

In some aspects, following a method of treatment disclosed herein, a subject's best corrected visual acuity (BCVA) improves by 1, 2 3, 4, 5 or more lines.

In some aspects, following a method of treatment disclosed herein, a reduction in neovascularization as assessed by Fluorscein Angiography (FA) occurs.

In some cases, retinal thickness may be measured to examine the effects of treatment. In some cases, following a method of treatment disclosed herein, the central retinal thickness of the human subject does not increase by more than 50 microns, 100 microns, or 250 microns within 12 months following treatment with the pharmaceutical composition of the disclosure. In some cases, following a method of treatment disclosed herein, the central retinal thickness of the human subject decreases by at least 50 microns, 100 microns, 200 microns, 250 microns, 300 microns, 400 microns, 500 microns, 600 microns within 3 months, 6 months or 9 months 12 months following treatment with the pharmaceutical composition of the disclosure. The decrease in the central retinal thickness of the human subject may be measured comparing the central retinal thickness at point in time to a baseline measurement taken at or within 1, 3, 7 or 10 days of the administration of the pharmaceutical composition of the disclosure.

Compositions and reagents useful for the present disclosure may be packaged in kits to facilitate application of the present disclosure. In some aspects, the present method provides for a kit comprising a virus or viral vector disclosed herein, e.g., a recombinant virus of the disclosure. In some embodiments, the kit comprises instructions for using the virus or viral vector, e.g., instructions on how to administer the virus or viral vector to a subject to treat an ocular disease or disorder. The instructions could be in any desired form, including but not limited to, printed on a kit insert, printed on one or more containers, as well as electronically stored instructions provided on an electronic storage medium, such as a computer readable storage medium. Also optionally included is a software package on a computer readable storage medium that permits the user to integrate the information and calculate a control dose.

In one aspect, a kit comprises one or more containers, each container comprising: (a) a unit dose of a pharmaceutical composition described herein, which comprises a recombinant virus provided herein (e.g., a rAAV), and (b) instructions on how to administer to a subject a therapeutically effective amount of the recombinant virus.

In some embodiments, the kit comprises two containers, wherein each container comprises a unit dose of a pharmaceutical composition described herein. In certain embodiments, the unit dose in each of the two containers is the same, whereas in other embodiments, the unit dose in each of the two containers is different. In particular embodiments, both of the two containers comprises a unit dose of at least about 1×10¹⁰ vg, at least about 1×10¹¹ vg, at least about 5×10¹¹ vg, at least about 1×10¹² vg, at least about 5×10¹² vg, at least about 1 × 10¹³ vg, at least about 5×10¹³ vg, or at least about 1×10¹⁴ vg. In some embodiments, one container for the first dose comprises about 1×10¹¹ vg up to about 5×10¹² vg, or either about 1×10¹⁰ vg up to about 1×10¹⁴ vg or about 1×10¹¹ vg up to about 1×10¹⁴ vg. In some embodiments, the second container for the second dose comprises about 1×10¹¹ vg up to about 5×10¹² vg, or either about 1×10¹¹ vg up to about 1×10¹⁴ vg or about 1×10¹¹ vg up to about 1×10¹⁴ vg. In some embodiments, the first container comprises at least about 1×10¹⁰ vg, at least about 1×10¹¹ vg, at least about 5×10¹¹ vg, at least about 1×10¹² vg, at least about 5×10¹² vg, or at least about 1 × 10¹³ vg. In some embodiments, the second container comprises at least about 5×10¹² vg, at least about 1×10¹³ vg, at least about 5×10¹³ vg, at least about 1×10¹⁴ vg, at least about 5×10¹⁴ vg, or at least about 1×10¹⁵ vg. In some embodiments, any unit dose comprises about 1×10¹² vg to about 5×10¹⁴ vg, or about 5×10¹² vg to about 1×10¹⁵ vg.

In some aspects, the kit may comprise pharmaceutically acceptable salts or solutions for administering the recombinant virus. Optionally, the kit can further comprise instructions for suitable operational parameters in the form of a label or a separate insert. For example, the kit may have standard instructions informing a physician or laboratory technician to prepare a dose of recombinant virus.

Optionally, the kit could further comprise devices for administration, such as a syringe, filter needle, extension tubing, cannula, and subretinal injector.

### EXAMPLES

### EXAMPLE 1

### Evaluating the Effect of nAbs on AAV2.7m8 Transgene Transduction in Vitro

Disclosed herein as an exemplary example is an in vitro assay for determination of the effect of nAbs on AAV2.7m8 transgene expression.

To detect the effect of increasing AAV2.7m8 titers on neutralization, an in vitro IVIg (Intravenous Immunoglobulin) assay was performed. Virus with multiplicities of infections (MOIs) in half-log increments from 1×10⁵ to 5 ×10⁶ were mixed in a 1:1 ratio with a 3-fold dilution series from 1:3-1:1000 of IVIg for 1 hour and added to 293T cells.

These were incubated for 72 hours followed by assessment of % GFP inhibition as a measure of nAb evasion. FIG. 1 depicts a plot of%GFP inhibition as a function of dilution of nAbs and as a function of the amount of viral MOI. This plot was used to calculate IC50 values. Table 1 depicts the IC50 values.

**Table 1: IC50 values of nAbs against AAV2.7m8-GFP transduction**

| Dose | 1E5 | 5E5 | 1E6 | 5E6 |
|---|---|---|---|---|
| **IC₅₀** | 240.1 | 44.53 | 21.98 | < 3.1 |

The nAb titer (IC50 values) reduced as the amount of virus was increased.

### Example 2

### Evaluating the Effect of nAbs on AAV2.7m8 Transgene Transduction in Vivo

Disclosed herein as an exemplary example is a study in NHPs to evaluate the immune response to the AAV2.7m8 vector in a staged manner. FIG. 2 describes the overall study design.

NHPs were used to model potentially relevant clinical conditions. Varying levels of IVIg and AAV2.7m8 doses were tested to simulate the effect of pre-existing nAbs on IVT injected AAV2.7m8. African green monkeys (AGM) were pre-screened for nAbs to AAV2.7m8 prior to study enrollment.

Fluorescence imaging was performed at weeks 2, 4, 8, 12 to assess GFP expression, as a measure of nAb evasion.

FIGS. 3A-3H depict exemplary Heidelberg Spectralis images of AGM retina at 4 weeks. FIG. 3A depicts an AGM receiving an intravitreal (IVT) IVIg injection (1.85 mg/mL) followed by a dose of IVT 7m8.CMV-GFP at 1 ×10¹¹ vg 24 hours later(Group 1). FIG. 3B depicts an AGM receiving an intravitreal injection of mouse monoclonal α-AAV2 (5 µg/mL) followed by an IVT dose of 7m8.CMV-GFP at 1 ×10¹¹ vg 24 hours later (Group 2). FIG. 3C depicts an AGM receiving an intravitreal injection of vehicle (50 µL) with an IVT dose of 7m8.CMV-GFP at 1 ×10¹¹ vg (Group 3). FIG. 3D depicts an AGM receiving an intravitreal injection of IVIg (0.46 mg/mL) followed by a dose of 7m8.CMV-GFP at 1 ×10¹¹ vg 24 hours later (Group 4). FIG. 3E depicts an AGM receiving an intravitreal injection of IVIg (1.85 mg/mL) followed by an IVT dose of 7m8.CMV-GFP at 5 ×10¹¹ vg 24 hours later (Group 5). FIG. 3F depicts an AGM receiving an intravitreal injection of mouse monoclonal α-AAV2 (5 µg/mL) followed by an IVT dose of 7m8.CMV-GFP at 5 ×10¹¹ vg 24 hours later (Group 6). FIG. 3G depicts an AGM receiving an intravitreal injection of vehicle (50 µL) followed by an IVT dose of 7m8.CMV-GFP at 5 ×10¹¹ vg 24 hour later (Group 7). FIG. 3H depicts an AGM receiving an intravitreal injection of IVIg (1.85 mg/mL) followed by an IVT dose of 7m8.CMV-GFP at 1 ×10¹³ vg 24 hours later (Group 8).

In the presence of high IVIg (1.85 mg/mL), 1 ×10¹¹ vg of AAV2.7m8 was neutralized and no GFP expression was observed (Group1).

When the amount of IVIg was 4-fold lower (0.46 mg/mL) in the eye, AAV2.7m8 at the 1×10¹¹ vg dose showed good GFP expression, which suggested nAb evasion (Group 4).

In the presence of high IVIg, a 5-fold higher vector dose (5×10¹¹ vg) showed no transduction of GFP (Group 5).

At a dose of 1 × 10¹³ vg of AAV2.7m8, GFP was expressed in the presence of a dose of high IVIg (1.85 mg/mL). This suggested that this amount of vector may overcome neutralization and can transduce cells (Group 8).

Monoclonal antibody to AAV2 neutralized AAV2.7m8 at 1 X 10¹¹ vg as well as 5 X 10¹¹ vg (Groups 2 and 6).

Serum, aqueous, and vitreous samples were collected to evaluate whether there was a correlation between nAbs levels in serum versus aqueous and vitreous. To determine nAb levels in the serum, aqueous, and vitreous of the study NHPs, an *in vitro* nAb assay was performed. AAV2.7m8 vector was mixed with serially diluted samples and incubated for an hour. This mixture was added to 293T cells at an MOI of 1 ×10⁵ and the transduced cells were cultured for 72 hrs. GFP expression was assessed on a plate reader to evaluate % GFP inhibition and calculate the IC50 values. Table 2 depicts an exemplary IC50 value table.

**Table 2: Exemplary in vitro IC50 values**

| Treatment | Animal | Serum nAbs | | Aqueous nAbs | | Vitreous nAbs | | Neutralization |
|---|---|---|---|---|---|---|---|---|
| | | Baseline | 12-wk | Baseline | 12-wk | Baseline | 12-wk | |
| | 562R | BLD | 1:10 | BLD | BLO | BLO | BLD | no |
| **1E11** vg/ml | 562L | | | BLD | BLD | BLD | BLD | no |
| | 586L | BLD | 1:3 | BLD | BlD | BLD | BLD | no |
| Low IVIG # 1E11vg/ml | 284R | BLD | 1:20 | BLD | BLD | BLD | BLD | no |
| | 284L | | | BLD | BLD | BLD | BLD | no |
| | 512R | BLD | 1:40 | BLD | BLD | BLD | 1:35 | no |
| | 512L | | | BLD | 1:30·1:90 | BlD | 1:200 | no |
| High IVIG * 5E11 vg/ml | 523R | BLD | BLD | BLD | BLD | BLD | BLD | yes |
| | 523L | | | BLD | BLD | BLD | BLD | yes |
| α-AAV2 + 5E11vg/ml | 548R | BLD | BlD | BLD | BLD | BLD | BLO | yes |
| | 548L | | | BLD | BLO | BLD | BLD | yes |
| 5E11 vg/ml | 522R | BLD | 1:20 | BLD | BLD | BLD | 1:25 | no |
| | 522L | | | BLD | 1:10·1:30 | BLD | 1:15 | no |
| | 560R | BLD | BLD | BLD | BLD | BLD | BLD | no |
| | 560L | | | 8LD | BLD | BLD | BLD | no |

| | | | | | | | | |
|---|---|---|---|---|---|---|---|---|
| BLD = Below Limit of Detection | | | | | | | | |

The invention is further characterized by the following items:
1. A method for providing a transgene encoding a gene product to an ocular cell in a subject, comprising administering to one or more sites within the eye of the subject an effective amount of a recombinant adeno-associated virus (rAAV) comprising said transgene, wherein the subject comprises neutralizing antibodies that specifically bind the rAAV, wherein the effective amount is an amount sufficient to at least partially evade the nAbs in the subject and transduce the ocular cell, and wherein the transduced ocular cell expresses the gene product.
2. The method of item 1, wherein the rAAV is an AAV2.7m8.
3. The method of item 1 or item 2, wherein the subject is a mammalian subject.
4. The method of any of items 1-3, wherein the effective amount is at least about 1×10¹¹ vg.
5. The method of any of items 1-3, wherein the effective amount is at least about 5×10¹¹ vg.
6. The method of any of items 1-3, wherein the effective amount is at least about 1×10¹³ vg.
7. The method of any of items 1-6, wherein the subject comprises neutralizing antibodies at a concentration between about 0.46 mg/mL to about 1.85 mg/mL at one or more of the one or more site of administration.
8. The method of any of items 1-7, wherein the ocular cell is a retinal cell.
9. The method of item 8, wherein the rAAV is administered retinally, subretinally, and/or intravitreally.
10. A method for treating an ocular disease or disorder in a subject in need thereof, comprising administering to one or more sites within the eye of the subject an effective amount of a recombinant adeno-associated virus (rAAV) comprising a transgene encoding a therapeutic gene product, wherein the subject comprises neutralizing antibodies that specifically bind the rAAV, wherein the effective amount is an amount sufficient to at least partially evade the nAbs in the subject and transduce ocular cells within the subject, and wherein the transduced ocular cells expresses the therapeutic gene product.
11. The method of item 10, wherein the rAAV is an AAV2.7m8.
12. The method of item 10 or item 11, wherein the subject is a mammalian subject.
13. The method of any of items 10-12, wherein the effective amount is at least about 1×10¹¹ vg.
14. The method of any of items 10-12, wherein the effective amount is at least about 5×10¹¹ vg.
15. The method of any of items 10-12, wherein the effective amount is at least about 1×10¹³ vg.
16. The method of any of items 10-15, wherein the subject comprises neutralizing antibodies at a concentration between about 0.46 mg/mL to about 1.85 mg/mL at one or more of the one or more site of administration.
17. The method of any of items 10-16, wherein the ocular cells are retinal cells.
18. The method of any of items 10-17, wherein the rAAV is administered retinally, subretinally, and/or intravitreally.
19. The method of any of items 10-18, wherein the ocular disease or disorder is glaucoma, retinitis pigmentosa, macular degeneration, retinoschisis, Leber's Congenital Amaurosis, diabetic retinopathy, achromotopsia, or color blindness.
20. The method of item 19, wherein the ocular disease is macular degeneration.
21. The method of item 20, wherein the macular degeneration is wet macular degeneration.
22. The method of item 20, wherein the macular degeneration is dry macular degeneration.
23. The method of any of items 10-22, wherein the gene product is an anti-angiogenic polypeptide, a vascular endothelial growth factor (VEGF)-binding protein, or an opsin protein.
24. A method for treating an ocular disease or disorder in a subject in need thereof, the method comprising:
   (a) administering to a first eye of the subject a first effective amount of a first recombinant adeno-associated virus (rAAV) comprising a first transgene encoding a first gene product;
   (b) waiting for a period of time; and
   (c) administering to a second eye of the subject a second effective amount of a second rAAV comprising a second transgene encoding a second gene product,
   wherein the first and second effective amounts are amounts sufficient to transduce cells of the eye, and wherein the transduced cells express the first and second gene products.
25. The method of item 24, wherein the first rAAV and the second rAAV are the same serotype.
26. The method of item 24 or item 25, wherein the first rAAV and the second rAAV comprise the same capsid proteins.
27. The method of any of items 24-26, wherein the first rAAV and the second rAAV are AAV2.7m8.
28. The method of item 24, wherein the first rAAV and the second rAAV are different serotypes.
29. The method of item 27 or item 28, wherein the first rAAV and the second rAAV comprise different serotypes.
30. The method of any of items 24-29, wherein the period of time is selected from the following: at least one week, at least one month, at least three months, at least six months, at least one year, at least 18 months, at least two years, at least three years, or longer than three years.
31. The method of any of items 24-30, wherein the subject is not administered a recombinant rAAV during the period of time.
32. The method of any of items 24-31, wherein the first and second gene products are the same.
33. The method of any of items 24-31, wherein the first and second gene product are different.
34. The method of any of items 24-33, wherein the first and/or second gene product is a therapeutic gene product.
35. The method of item 34, wherein the first gene product and the second gene product are independently selected from: an anti-angiogenic polypeptide, a vascular endothelial growth factor (VEGF)-binding protein, an anti-VEGF agent, or an opsin protein.
36. The method of any of items 24-35, wherein the ocular disease or disorder is glaucoma, retinitis pigmentosa, macular degeneration, retinoschisis, Leber's Congenital Amaurosis, diabetic retinopathy, achromotopsia, or color blindness.
37. The method of item 36, wherein the ocular disease is macular degeneration.
38. The method of item 37, wherein the macular degeneration is wet macular degeneration.
39. The method of item 37, wherein the macular degeneration is dry macular degeneration.
40. The method of any of items 24-39, wherein the first effective amount is at least about 1×10¹¹ vg.
41. The method of any of items 24-39, wherein the first effective amount is at least about 5×10¹¹ vg.
42. The method of any of items 24-29, wherein the first effective amount is at least about 1×10¹³ vg.
43. The method of any of items 24-42, wherein the second effective amount is at least about 1×10¹¹ vg.
44. The method of any of items 24-42, wherein the second effective amount is at least about 5×10¹¹ vg.
45. The method of any of items 24-42, wherein the second effective amount is at least about 1×10¹³ vg.
46. The method of any of items 24-42, wherein the first effective amount comprises up to about 1 × 10¹¹ vector genomes of the first rAAV.
47. The method of any of items 24-42, wherein the first effective amount comprises between about 1 × 10¹¹ and 5 × 10¹¹ vector genomes of the first rAAV.
48. The method of any of items 24-42, wherein the first effective amount comprises up to about 1 × 10¹¹ vector genomes of the first rAAV, and the second effective amount comprises at least about 1 × 10¹¹ vector genomes of the second rAAV.
49. The method of any of items 24-48, wherein following the time period, the subject comprises neutralizing antibodies that specifically bind the first rAAV, wherein the second effective amount is an amount sufficient to at least partially evade the nAbs in the subject and transduce the ocular cell, and wherein the transduced ocular cell expresses the gene product.
50. The method of any of items 24-49, wherein the first effective amount is lower than the second effective amount.
52. The method of any of items 24-50, wherein the subject is not administered an immunosuppressant prior to, concurrent with, or following administration of the first rAAV.
52. The method of any of items 24-50, wherein the subject is administered an immunosuppressant prior to, concurrent with, or following administration of the first rAAV.
53. The method of any of items 24-50, wherein an immunosuppressant is not administered to the subject after the administration of the first rAAV.
54. The method of any of items 24-50, wherein an immunosuppressant is administered to the subject after the administration of the first rAAV and before or concurrent with the administration of the second rAAV.
55. The method of any of items 24-50, wherein an immunosuppressant is administered to the subject after the administration of the second rAAV.
56. The method of any of items 24-55, wherein the administering of the first effective amount and the administering of the second effective amount is by intraocular injection or intravitreal injection.
57. The method of any of items 24-56, wherein administration of the second effective amount of the second rAAV provides a therapeutic effect in the eye of the subject.
58. The method of any of items 24-57, wherein the first effective amount and the second effective amount are administered to the same eye of the subject.
59. The method of any of items 24-57, wherein the first effective amount and the second effective amount are administered to different eyes of the subject.
60. The method of any of items 24-59, wherein the first effective amount and the second effective amount is treating the same ocular disease or disorder.
61. The method of any of items 24-59, wherein the first effective amount and the second effective amount is treating different ocular diseases or disorders.
62. The method of any of items 24-61, wherein the first gene product and the second gene product are the same.
63. The method of any of items 24-61, wherein the first gene product and the second gene product are different.

## Claims

1. A recombinant adeno-associated virus (rAAV) comprising a transgene for use in a method for providing a transgene encoding a gene product to an ocular cell in a subject, comprising
(a) administering to one or more sites within the eye of the subject an effective amount of a recombinant adeno-associated virus (rAAV) comprising said transgene, wherein the subject comprises neutralizing antibodies that specifically bind the rAAV, wherein the effective amount is an amount sufficient to at least partially evade the nAbs in the subject and transduce the ocular cell, and wherein the transduced ocular cell expresses the gene product.

2. The recombinant adeno-associated virus (rAAV) comprising a transgene for use of claim 1,
(a) wherein the rAAV is an AAV2.7m8;
(b) wherein the subject is a mammalian subject;
(c) wherein the ocular cell is a retinal cell; and/or
(d) wherein the rAAV is administered retinally, subretinally, and/or intravitreally.

3. The recombinant adeno-associated virus (rAAV) comprising a transgene for use of claim 1 or 2, wherein the effective amount is at least about 1×10¹¹ vg preferably at least about 5×10¹¹ vg, more preferably at least about 1×10¹³ vg.

4. The recombinant adeno-associated virus (rAAV) comprising a transgene for use of any of claims 1-3, wherein the subject comprises neutralizing antibodies at a concentration between about 0.46 mg/mL to about 1.85 mg/mL at one or more of the one or more site of administration.

5. The recombinant adeno-associated virus (rAAV) comprising a transgene for use of claim 1, wherein the method for providing said transgene encoding a gene product to an ocular cell is for treating an ocular disease or disorder in a subject in need thereof and wherein the transgene encodes a therapeutic gene product.

6. The recombinant adeno-associated virus (rAAV) comprising a transgene for use of claim 5,
(a) wherein the rAAV is an AAV2.7m8;
(b) wherein the subject is a mammalian subject;
(c) wherein the effective amount is at least about 1×10¹¹ vg, preferably at least about 5×10¹¹ vg, more preferably at least about 1×10¹³ vg;
(d) wherein the subject comprises neutralizing antibodies at a concentration between about 0.46 mg/mL to about 1.85 mg/mL at one or more of the one or more site of administration;
(e) wherein the ocular cells are retinal cells;
(f) wherein the rAAV is administered retinally, subretinally, and/or intravitreally;
(g) wherein the ocular disease or disorder is glaucoma, retinitis pigmentosa, macular degeneration, retinoschisis, Leber's Congenital Amaurosis, diabetic retinopathy, achromotopsia, or color blindness, preferably wherein the ocular disease is macular degeneration, more preferably wherein the macular degeneration is wet macular degeneration or dry macular degeneration; and/or
(h) wherein the gene product is an anti-angiogenic polypeptide, a vascular endothelial growth factor (VEGF)-binding protein, or an opsin protein.

7. The recombinant adeno-associated virus (rAAV) comprising a transgene for use of claim 1, wherein the method for providing said transgene encoding a gene product to an ocular cell is for treating an ocular disease or disorder in a subject in need thereof, the method further comprising:
(b) waiting for a period of time; and
(c) administering to a second eye of the subject a second effective amount of a second rAAV comprising a second transgene encoding a second gene product,
wherein the first and second effective amounts are amounts sufficient to transduce cells of the eye, and wherein the transduced cells express the first and second gene products.

8. The recombinant adeno-associated virus (rAAV) comprising a transgene for use of claim 7,
(a) wherein the first rAAV and the second rAAV are the same serotype;
(b) wherein the first rAAV and the second rAAV comprise the same capsid proteins;
(c) wherein the first rAAV and the second rAAV are AAV2.7m8;
(d) wherein the first rAAV and the second rAAV are different serotypes; or
(e) wherein the first rAAV and the second rAAV comprise different capsid proteins.

9. The recombinant adeno-associated virus (rAAV) comprising a transgene for use of claim 7 or 8,
(a) wherein the period of time is selected from the following: at least one week, at least one month, at least three months, at least six months, at least one year, at least 18 months, at least two years, at least three years, or longer than three years; and/or
(b) wherein the subject is not administered a recombinant rAAV during the period of time.

10. The recombinant adeno-associated virus (rAAV) comprising a transgene for use of any of claims 7-9, wherein the first and second gene products are the same or wherein the first and second gene product are different.

11. The recombinant adeno-associated virus (rAAV) comprising a transgene for use of any of claims 7-10, wherein the first and/or second gene product is a therapeutic gene product, preferably wherein the first gene product and the second gene product are independently selected from: an anti-angiogenic polypeptide, a vascular endothelial growth factor (VEGF)-binding protein, an anti-VEGF agent, or an opsin protein.

12. The recombinant adeno-associated virus (rAAV) comprising a transgene for use of any of claims 7-11, wherein the ocular disease or disorder is glaucoma, retinitis pigmentosa, macular degeneration, retinoschisis, Leber's Congenital Amaurosis, diabetic retinopathy, achromotopsia, or color blindness, preferably wherein the ocular disease is macular degeneration, more preferably wet macular degeneration, or dry macular degeneration.

13. The recombinant adeno-associated virus (rAAV) comprising a transgene for use of any of claims 7-12,
(a) wherein the first effective amount is at least about 1×10¹¹vg, preferably at least about 5×10¹¹vg, more preferably at least about 1×10¹³ vg;
(b) wherein the second effective amount is at least about 1×10¹¹vg, preferably at least about 5×10¹¹vg, more preferably at least about 1×10¹³ vg;
(c) wherein the first effective amount comprises up to about 1 × 10¹¹ vector genomes of the first rAAV;
(d) wherein the first effective amount comprises between about 1 × 10¹¹ and 5 × 10¹¹ vector genomes of the first rAAV;
(e) wherein the first effective amount comprises up to about 1 × 10¹¹ vector genomes of the first rAAV, and the second effective amount comprises at least about 1 × 10¹¹ vector genomes of the second rAAV;
(f) wherein following the time period, the subject comprises neutralizing antibodies that specifically bind the first rAAV, wherein the second effective amount is an amount sufficient to at least partially evade the nAbs in the subject and transduce the ocular cell, and wherein the transduced ocular cell expresses the gene product; and/or
(g) wherein the first effective amount is lower than the second effective amount.

14. The recombinant adeno-associated virus (rAAV) comprising a transgene for use of any of claims 7-13,
(a) wherein the subject is not administered an immunosuppressant prior to, concurrent with, or following administration of the first rAAV;
(b) wherein the subject is administered an immunosuppressant prior to, concurrent with, or following administration of the first rAAV;
(c) wherein an immunosuppressant is not administered to the subject after the administration of the first rAAV;
(d) wherein an immunosuppressant is administered to the subject after the administration of the first rAAV and before or concurrent with the administration of the second rAAV; and/or
(e) wherein an immunosuppressant is administered to the subject after the administration of the second rAAV.

15. The recombinant adeno-associated virus (rAAV) comprising a transgene for use of any of claims 7-14,
(a) wherein the administering of the first effective amount and the administering of the second effective amount is by intraocular injection or intravitreal injection;
(b) wherein administration of the second effective amount of the second rAAV provides a therapeutic effect in the eye of the subject;
(c) wherein the first effective amount and the second effective amount are administered to the same eye of the subject;
(d) wherein the first effective amount and the second effective amount are administered to different eyes of the subject;
(e) wherein the first effective amount and the second effective amount is treating the same ocular disease or disorder;
(f) wherein the first effective amount and the second effective amount is treating different ocular diseases or disorders;
(g) wherein the first gene product and the second gene product are the same; and/or
(h) wherein the first gene product and the second gene product are different.
